# EUROPEAN PATENT SPECIFICATION

(11) **EP 4 350 328 B1**
(45) Date of publication and mention of the grant of the patent: **11.11.2026**
(21) Application number: 23202186.5
(22) Date of filing: 06.10.2023
(51) Int. Cl.: G01N 19/04, G01N 13/00, G01N 33/00

(54) **METHOD FOR DETERMINING ADHESABILITY OF A FILM**
VERFAHREN ZUR BESTIMMUNG DER VERKLEBBARKEIT EINES FILMS
PROCÉDÉ DE DÉTERMINATION DE L'ADHÉSIVITÉ D'UN FILM

(30) Priority: 06.10.2022 EP 22199932
(43) Date of publication of application: 10.04.2024
(73) Proprietor: The Procter & Gamble Company, Cincinnati, OH 45202 (US)
(72) Inventor: ZONFRILLI, Fabio, 1853 Strombeek-Bever, Brussels (BE); GABRIELE, Andrea, 1853 Strombeek-Bever (BE)
(74) Representative: P&G Patent Belgium UK

(56) References cited:
- CN-A- 112 578 085
- V. NOZAHIC ET AL: "Influence of sunflower aggregates surface treatments on physical properties and adhesion with a mineral binder", COMPOSITES PART A: APPLIED SCIENCE AND MANUFACTURING, vol. 43, no. 11, 1 November 2012 (2012-11-01), pages 1837 - 1849, XP055194685, ISSN: 1359-835X, DOI: 10.1016/j.compositesa.2012.07.011
- FERREIRA ET AL: "Development of a new photocrosslinkable biodegradable bioadhesive", INTERNATIONAL JOURNAL OF PHARMACEUTICS, ELSEVIER, NL, vol. 352, no. 1-2, 11 December 2007 (2007-12-11), pages 172 - 181, XP022495274, ISSN: 0378-5173, DOI: 10.1016/J.IJPHARM.2007.10.026

## Description

### Field of the invention

The present invention is in a field of wet adhesion films, and methods, systems and computer programs for measurement of the film adhesability.

### Background to the invention

Wet adhesion films are used as packaging for different substances including detergents, food products, pharmaceuticals and agricultural chemicals. Typically a portion of the film is wetted and the wetted portion is brought into contact with another substrate, thereby forming a joint between the film and substrate in order to form a part of a product that is container or pouch for holding the substance.

Manufacture of a wet-adhesion film need to be carefully controlled so that the film acquires adhesivity within a certain time-frame after wetting. If adhesability is not reached, the parts of the joint will be brought together too soon during manufacturing of the product, the joint will be weak, and the container or pouch can fail during subsequent storage and/or transport. Batch to batch film variability can lead to films that acquire adhesivity at different times, some outside a tolerated range. Currently, the strength of the joint is tested in the product joint, however, no method exists for measuring adhesability of the film prior to formation of the joint in a product.

Patent application CN112578085 discloses coating a slurry containing a binder composition into a coated diaphragm and soaking in an electrolyte, and then testing the peel strength of the coated diaphragm before and after soaking to judge the bonding performance of the adhesive, and testing the coated diaphragm before and after soaking.

The present disclosures provides methods, computer programs and systems for measuring adhesability of films.

### Summary of the invention

Provided here is a method for determining an adhesability of a film sample (108), wherein adhesability is a prediction of bonding strength between the film sample and a substrate, wherein the film sample (108) is a sample of a wet-adhesion film, said film sample (108) having a first surface (106), an opposing second surface (104) and a straight side edge (102) connecting the first surface (106) and the second surface (104), comprising:
- contacting the straight side edge (102) of the film sample with an aqueous liquid (160),
- measuring, from the film sample (108), a set of expansion parameters comprising:
   - a swelling rate of the film sample (108), and
   - a penetration rate of the aqueous liquid (160) into the film sample (108) resulting from the contacting,
- determining from the expansion parameters, the adhesability of the film sample (108).

Preferably the set of expansion parameters is measured comprising the steps:
- generating an image dataset comprising a plurality of optical images (200, a,b,c) of at least a portion of the straight side edge (102), each optical image (200, a, b) captured at a different time point during the contacting;
- identifying in each optical image (200, a,b,c) or from an image portion thereof (200', a,b,c) a first front edge (202, a,b,c) corresponding to the straight side edge (102), and a second front edge (262, a,b,c) corresponding to a front edge (162) of the aqueous liquid (160) penetrating the film sample (108); and

- the swelling rate is determined from a position (*p1*) of the first front edge (202, a,b,c) over the different timepoints, and the penetration rate is determined from a position (*p2*) of the second front edge (262a,b,c) over the different timepoints.

Preferably, the first (202, a,b) and second (262, a,b) front-edges are identified comprising the steps:
- for each optical image (200, a,b) in the image dataset, or image portion thereof (200', a,b):
   - with the first front edge (202, a,b,c) aligned parallel to a y-axis, and an x-axis being perpendicular to the y-axis;
   - applying a first filter to the optical image (200, a,b) or image portion (200',a,b), comprising replacing (C1' to C4') each pixel of each column (C1 to C4) of pixels parallel to the y-axis into an average of grey intensity for that column;
   - optionally applying one or more edge-enhancing filters to each previously-filtered image;
   - thereby obtaining an enhanced image (300, a,b) or enhanced image portion (300',a,b),
   - identifying from the enhanced image (300, a,b) or enhanced image portion (300',a,b) thereof, an enhanced first region (302, a,b) and enhanced second region (362,a,b), the enhanced first (302, a,b) and second (362, a,b,c) regions have the highest grey intensities compared with other regions, wherein the enhanced first region (302, a,b) corresponds to the first front edge (202, a,b) and the enhanced second region (362,a,b) corresponds to the second front edge (262, a,b).

Preferably, the identifying from enhanced image (300, a,b) the first front edge (222,a, b) and second front edge (262,a, b)) further comprises the steps:
- for each enhanced image (300, a,b) or enhanced image portion (300', a,b)
- determining an intensity profile (400,a, b) along the x-axis of the enhanced image (302,a,b), wherein intensity profile (400,a, b) indicates along one axis pixel intensity of the enhanced image (302,a,b) or enhanced image portion (300', a,b) as an amplitude, and along another axis position along the x-axis of the enhanced image (302,a,b) or enhanced image portion (300', a,b),
- optionally applying one or more noise-reducing filters to the intensity profile (400,a, b)
- identifying in the intensity profile (400a, b), optionally noise-filtered, a first peak (402,a,b) and a second peak (462,a,b) have the highest maximum amplitudes compared with other regions, wherein the first peak (402,a,b) corresponds to the first front edge (202, a,b) and the second peak (462,a,b) corresponds to the second front edge (262a,b,c).

Preferably, the adhesability is determined from the swelling rate and from the penetration rate, wherein a film sample having a higher adhesability has a higher swelling rate and penetration rate compared with a film sample having a lower adhesability.

Preferably, the determining from the expansion parameters, the adhesability of the film sample (108) comprises the steps:
- receiving as an input to a trained predictive model the set of expansion parameters,
- outputting from the trained predictive model the adhesability of the film sample (108), wherein the model has been trained using a training data set comprising swelling rate, penetration rate, and experimentally measured adhesability, and
- a training input to the predictive model is the swelling rate, the penetration rate, and a training output is predicted adhesability,
- the predicted adhesability during training is compared with the experimentally measured adhesability, and
- the predictive model is adjusted during training so that the outputted predicted adhesability approaches the experimentally measured adhesability of the training data set.

Preferably, the swelling rate and the penetration rate are compared with known swelling rates and known penetration rates of samples having known adhesabilities, thereby determining adhesability of the film sample (108).

Preferably:
- the swelling rate is represented as a first exponential function fitted to the position (*p1*) of the first front edge (202, a,b,c) over the different timepoints,
- the penetration rate is represented as a second exponential function fitted to the position (*p2*) of the second front edge (262, a,b,c) over the different timepoints,
wherein the first exponential function and the second exponential function are compared with known first and second exponential functions of samples having known adhesabilities, thereby determining adhesability of the film sample (108).

Preferably, the film is a wet adhesion film containing one or more of acrylonitrile butadiene styrene, polybutadiene, polycarbonates, polysulfones, polyethylene terephthalate, poly(methyl methacrylate), polystyrene, polyvinyl phloride, polyvinylalcohol-water, polyvinylalcohol; preferably containing polyvinyl alcohol. Preferably, the film is a wet adhesion film containing polyvinyl alcohol.

In a preferred embodiment the method according to the invention is a computer-implemented method for determining an adhesability of a film sample (108) wherein adhesability is a prediction of bonding strength between the film sample and a substrate, wherein the film sample (108) is a sample of a wet-adhesion film, the method comprising:
- receiving an image dataset comprising a plurality of optical images (200, a,b,c) of at least a portion of a straight side edge (102) of the film sample (108), each optical image (200, a,b,c) captured at a different time point during contacting the straight side edge (102) of the film sample (108) with an aqueous liquid (160),
- identifying in each optical image (200, a,b,c) or an image portion (200', a,b,c) thereof a first front edge (202, a,b,c) corresponding to the straight side edge (102), and a second front edge (262a,b,c) corresponding to a liquid front (162) of the aqueous liquid (160) penetrating the film sample (108); and
- determining a set of expansion parameters comprising:
   - a swelling rate of the film sample, and
   - a penetration rate of the aqueous liquid (160) into the film sample (108),
   wherein the swelling rate is determined from a change in position (*p1*) of the first front edge (202, a,b,c) over the different timepoints, and the penetration rate is determined from a change in position (*p2*) of the second front edge (262a,b,c) over the different timepoints,
- determining from the expansion parameters, the adhesability of the film sample (108).

The computer implemented method incorporating the subject matter of the method for determining an adhesability of a film sample described herein.

An unclaimed example useful to understand the present invention is a system comprising a processor adapted to perform the computer-implemented method described herein.

In a preferred embodiment the method according to the invention is a computer-implemented method for determining an adhesability of a film sample (108) wherein adhesability is a prediction of bonding strength between the film sample and a substrate, wherein the film sample (108) is a sample of a wet-adhesion film, the method comprising:
- sending, to a remote processor, an image dataset comprising a plurality of optical images (200, a,b,c) of at least a portion of a straight side edge (102) of the film sample (108), each optical image (200, a,b,c) captured at a different time point during contacting the straight side edge (102) of the film sample (108) with an aqueous liquid (160);
- receiving from the remote processor, the adhesability of the film sample, wherein the adhesability of the film sample has been determined by:
   - identifying in each optical image (200, a,b,c) a first front edge (202, a,b,c) corresponding to the straight side edge (102), and a second front edge (262a,b,c) corresponding to a liquid front (162) of the aqueous liquid (160) penetrating the film sample (108); and
   - determining a set of expansion parameters comprising:
      - a swelling rate of the film sample (108), and
      - a penetration rate of the aqueous liquid (160) into the film sample (108),
      wherein the swelling rate is determined from a change in position (*p1*) of the first front edge (202, a,b,c) over the different timepoints, and the penetration rate is determined from a change in position (*p2*) of the second front edge (262a,b,c) over the different timepoints,
   - determining from the expansion parameters, the adhesability of the film sample (108).

The computer implemented method incorporating the subject matter of the method for determining an adhesability of a film sample described herein. An unclaimed example useful to understand the present invention is a system comprising a processor adapted to perform the computer-implemented method described herein.

### Figure Legends

**FIG. 1A** is a three-dimensional schematic view of a film sample.
**FIG. 1B** is a plan schematic view of the film sample of FIG. 1A
**FIG. 2** is a schematic view the film sample and aqueous liquid prior to mutual contacting.

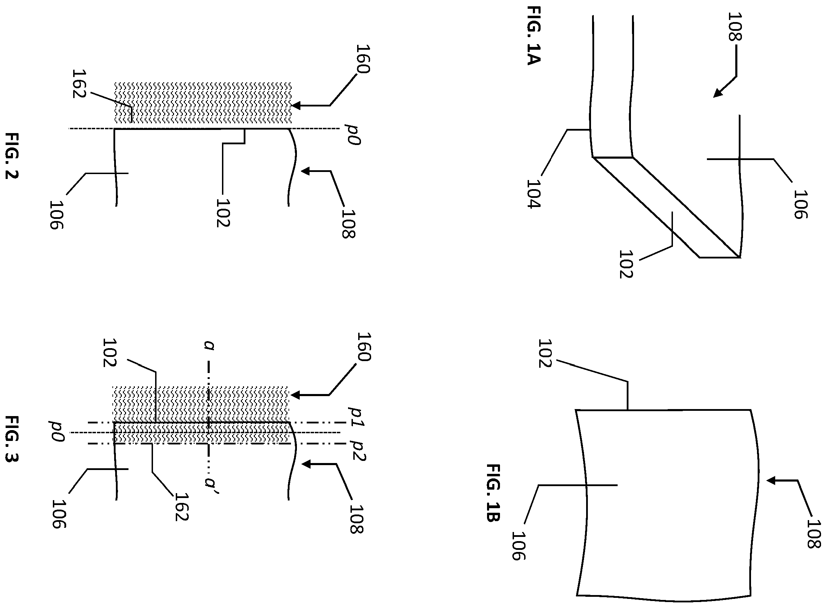

**FIG. 2** is a schematic view the film sample and aqueous liquid after mutual contacting, film swelling and liquid penetration.
**FIG. 3** is a schematic view the film sample and aqueous liquid after mutual contacting, film swelling and liquid penetration.

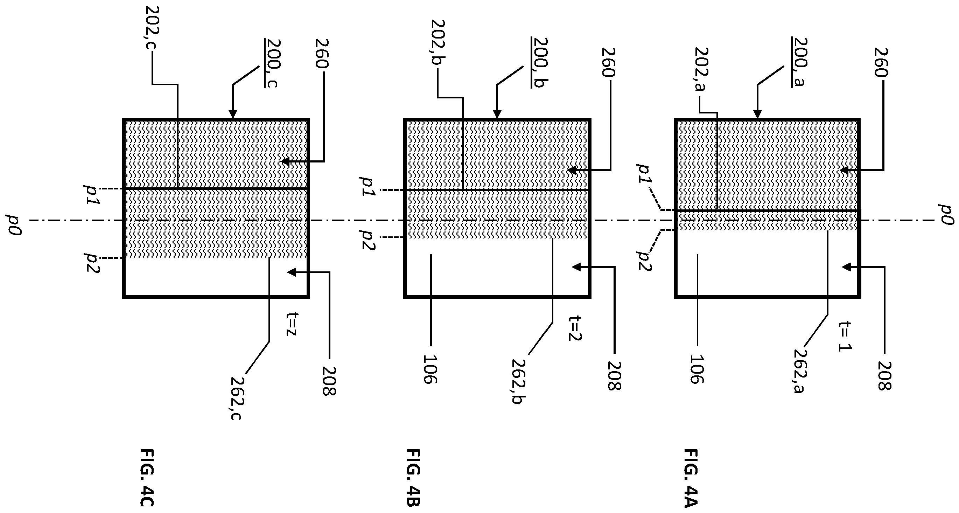

**FIGs. 4A** to **4C** are a schematic views of optical images and image portions there at different times during contacting (FIG. 4A t=1; FIG. 4B t=2; FIG. 4C t=z;).

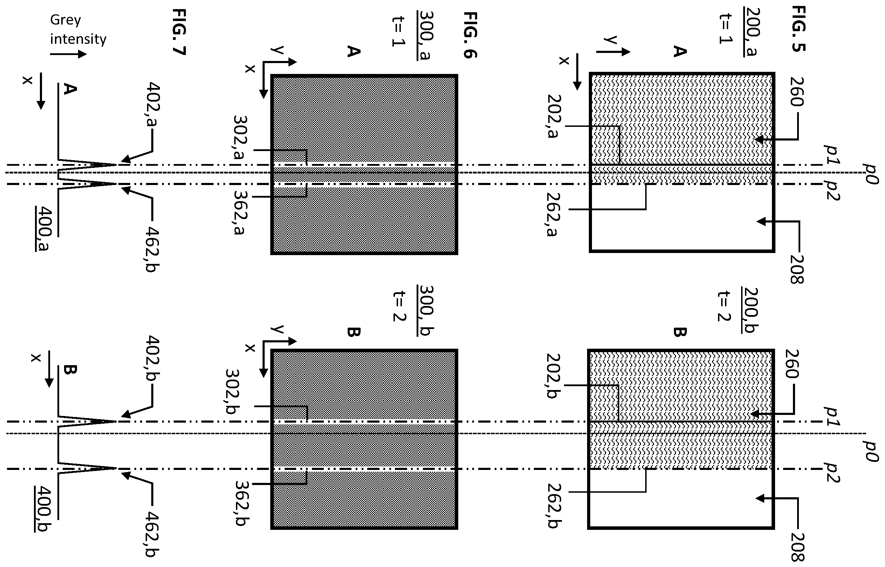

**FIGs. 5A** and **5B** are a schematic views of optical images at different times during contacting (FIG. 5A t=1; FIG. 5B t=2).
**FIGs. 6A** and **6B** are a schematic views of enhanced optical images at different times during contacting (FIG. 6A t=1; FIG. 6B t=2).
**FIGs. 7A** and **7B** are examples of intensity profiles at different times during contacting (FIG. 7A t=1; FIG. 7B t=2).

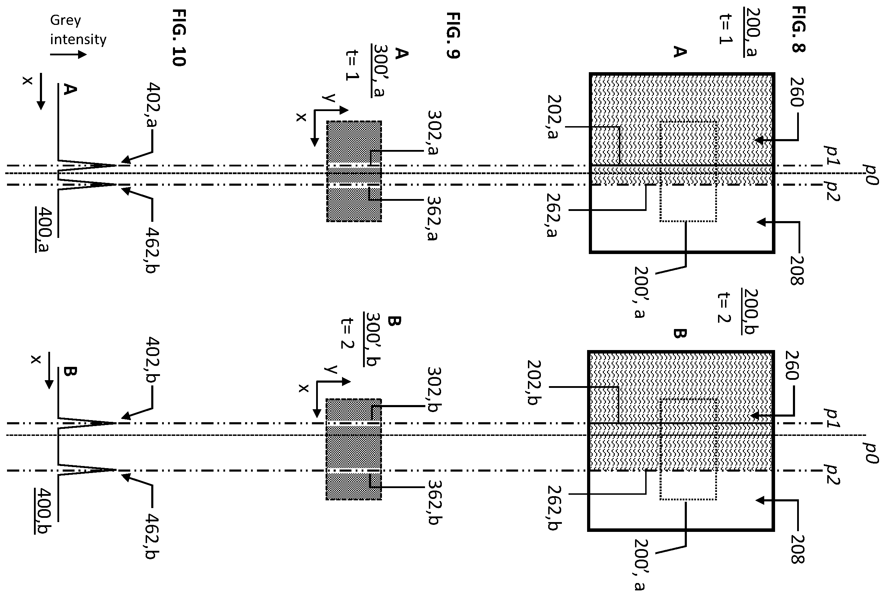

**FIGs. 8A** and **8B** are a schematic views of optical images and optical image portions thereof at different times during contacting (FIG. 5A t=1; FIG. 5B t=2).
**FIGs. 9A** and **9B** are a schematic views of optical image portions at different times during contacting (FIG. 6A t=1; FIG. 6B t=2).
**FIGs. 10A** and **10B** are examples of intensity profiles at different times during contacting (FIG. 7A t=1; FIG. 7B t=2).

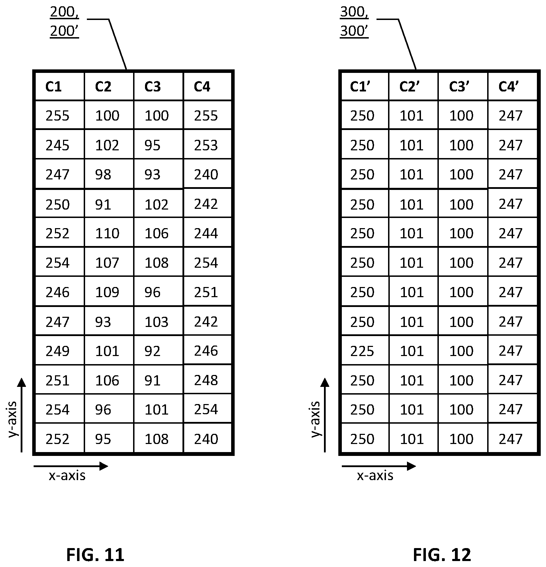

**FIG. 11** is a schematic pixel grey intensity representation of an optical image or optical image portion.
**FIG. 12** is a schematic pixel grey intensity representation of an enhanced optical image or enhanced optical image portion from averaging pixel column of FIG. 11.

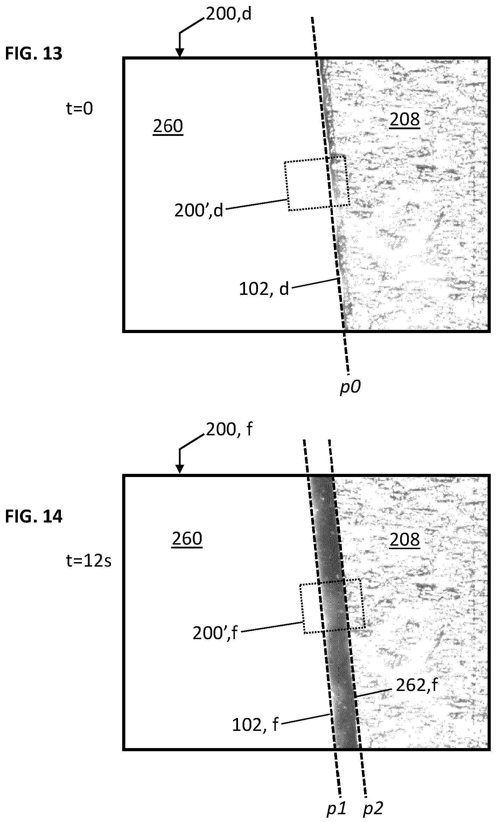

**FIG. 13** shows an optical image and optical image portion thereof of the film sample prior to contacting with aqueous liquid.
**FIG. 14** shows an optical image and optical image portion thereof of the film sample during contacting, film swelling and aqueous liquid penetration has completed.

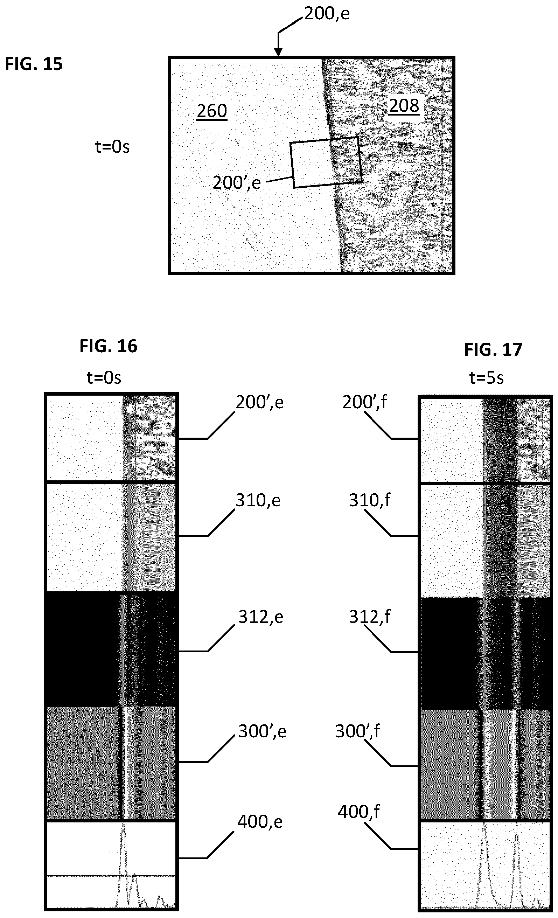

**FIG. 15** shows an optical image and optical image portion thereof of the film sample prior to contacting with aqueous liquid.
**FIG. 16** shows in alignment from top to bottom: optical image portion of the film sample of FIG 15, result of applying the first filter, result of subsequently applying an edge-enhancing (Sobel) filter, result of subsequently applying an edge-enhancing (Laplace) filter, an intensity profile.
**FIG. 17** shows in alignment from top to bottom: optical image portion of the film sample of FIG. 15/16 after contacting with aqueous liquid for 5 seconds, result of applying the first filter, result of subsequently applying an edge-enhancing (Sobel) filter, result of subsequently applying an edge-enhancing (Laplace) filter, an intensity profile wherein noise reducing filters have been applied to the intensity profile.

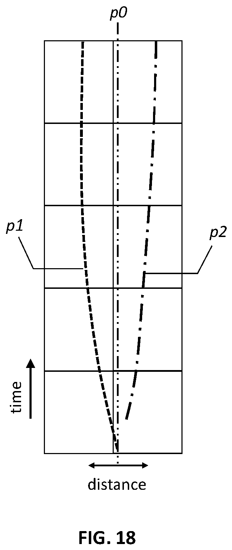

**FIG. 18** show a plot of position (*p1*) (first peak or straight side edge) and position (*p2*) (second peak or liquid front) at different time points.

### Detailed description of invention

Before the present methods, computer programs and systems of the invention are described, it is to be understood that this invention is not limited to particular methods, computer programs and systems or combinations described, since such methods, computer programs and systems and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention if they fall under the scope of the appended claims, and form different embodiments, as would be understood by those in the art.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. Unless otherwise indicated, all figures and drawings in this document are not to scale and are chosen for the purpose of illustrating different embodiments of the invention. In particular the dimensions of the various components are depicted in illustrative terms only, and no relationship between the dimensions of the various components should be inferred from the drawings, unless so indicated.

It is to be understood that other embodiments may be utilised and structural or logical changes may be made without departing from the scope of the present invention as defined by the appended claims. The following detailed description, therefore, is not to be taken in a limiting sense, and the scope of the present invention is defined by the appended claims.

The present invention concerns a method for determining an adhesability of a film sample. The film sample is a sample of a wet-adhesion film. An exemplary film sample (108) is depicted in **FIGs. 1A** and **1B****.** The film sample (108) has a first surface (106), an opposing second surface (104) and a straight side edge (102) connecting the first surface (106) and the second surface (104). The method comprises contacting the straight side edge (102) of the film sample with an aqueous liquid (160) and measuring, from the film sample (108), a set of expansion parameters. The expansion parameters comprise a swelling rate of the film sample (108), and a penetration rate of the aqueous liquid (160) into the film sample (108) resulting from the contacting. The adhesability of the film sample (108) is determined from the set of expansion parameters.

The adhesability of the film sample is a prediction of bonding strength between the film sample and a substrate. The substrate is typically another film of the same composition or another water-soluble film. The better (e.g. higher) the adhesability, the greater the predicted strength of the bond. In particular, the adhesability of the film sample may be a prediction of bonding strength within a product utilising the film. The product is typically a pouch or container for a substance, such as a liquid detergent, wherein the film forms a containing wall for holding the substance.

More in particular, the adhesability of the film sample may be a prediction of the bonding strength between the film sample and the substrate after wetting of the film sample for a defined time period. It allows a comparison in predicted bonding strength between different compositions of film that have been wetted for the same time period. The faster a level of adhesion is reached after wetting, the stronger the bond will be in a subsequent manufacturing process where film is contacted with the substrate for bonding after a defined time period following wetting.

Bonding strength in a product is currently measured according to a force needed cause the bond to fail in the product. However product (*e.g.* pouch) strength can be measured only after the full production process is executed, because measurement typically involves application of external mechanical pressure to the product. For instance, an external compressive mechanical force applied to a liquid filled pouch will exert a hydraulic force on the bond. The hydraulic force is representative of forces that might be applied in real-world situations to the product *e.g*. during storage or stacking. Standard mechanical test for capsule integrity requires it to resist a compression force 300N (see, for instance, Liquid Laundry Detergent Capsules Guidelines On CLP Implementation, Version 2.0, International Association for Soaps, Detergents and Maintenance Products, 26 November 2018). The mechanical compression test is different from other types of measurement of bonding strength such as a measurement of peeling force which is not representative of real-world stresses. Being able to predict bonding strength and hence product strength without involving the manufacturing process saves costs and time.

Further, there can be variability between different batches of film, which can result in poor bonding. The present method allows the film to be quality controlled in a location remote (*e.g.* at film manufacturer/supplier) from the product manufacturing facility.

The stronger the joint formed with a shorter wetting time, the more efficient is product manufacture; higher manufacturing throughput can be maintained because wetting times can be minimised. Ideally, a wetting time is between 1 and 4 seconds is a goal in an efficient manufacturing process. The present method may allow prediction of wetting time.

Adhesability may be expressed on a scale. For instance, on a scale of 0 (low adhesability, low predicted bonding strength) to 100 (high adhesability, high predicted bonding strength), or *vice versa.*

Preferably, adhesability is expressed as an indication of a pass (adhesability acceptable, acceptable bonding strength for the intended use) or fail (adhesability not acceptable, bonding strength not acceptable for the intended use).

The film sample (108) has a first surface (106), an opposing second surface (104). One surface (*e.g.* first surface (106)) may be smoother than the other (*e.g.* second surface (104)). The film sample (108) is preferably a single layer film.

The film sample is a sample (a portion) of a film. The film is a polymeric film. The film preferably comprises polyvinylalcohol (PVOH). The film is a wet-adhesion film. It has a wet adhesion property, namely, it can affix to a substrate material in the presence of aqueous liquid. The film is water soluble.

The film sample has a straight side edge (102) connecting the first surface (106) and the second surface (104). Other side edges of the film sample may or may not be straight. In order to achieve a straight side edge (102), an edge of the film may be cut in a straight line. The film may be cut using a blade to achieve the straight side edge (102).

The film preferably has a thickness of from 20 to 150 micron, preferably 35 to 125 micron, even more preferably 50 to 110 micron, most preferably about 76 micron.

By 'film' is it meant independently a first film layer, a second film layer if present, a third film layer if present, or a combination thereof in a multi-layer film arrangement. Preferably, the film contains only one film layer.

The film is soluble or dispersible in water. The film may be hot or cold-water soluble.

Preferred films exhibit good dissolution in cold water, meaning unheated distilled water. Preferably such films exhibit good dissolution at temperatures of 24°C, even more preferably at 10°C. By good dissolution it is meant that the film exhibits water-solubility of at least 50%, preferably at least 75% or even at least 95%, as measured by the method set out here after using a glass-filter with a maximum pore size of 20 microns, described above.

Preferably, the film has a water-solubility of at least 50%, preferably at least 75% or even at least 95%, as measured by the method set out here after using a glass-filter with a maximum pore size of 20 microns: 5 grams ± 0.1 gram of film material is added in a pre-weighed 3L beaker and 2L * 5ml of distilled water is added. This is stirred vigorously on a magnetic stirrer, Labline model No. 1250 or equivalent and 5 cm magnetic stirrer, set at 600 rpm, for 30 minutes at 30°C. Then, the mixture is filtered through a folded qualitative sintered-glass filter with a pore size as defined above (max. 20 micron). The water is dried off from the collected filtrate by any conventional method, and the weight of the remaining material is determined (which is the dissolved or dispersed fraction). Then, the percentage solubility or dispersability can be calculated.

The film may be obtained by casting, blow-moulding, extrusion or blown extrusion of the polymeric material, as known in the art.

The film may comprise one or more of acrylonitrile butadiene styrene (ABS), polybutadiene [butadiene rubber BR], polycarbonates (PC), polysulfones (PES), polyethylene terephthalate (PET), poly(methyl methacrylate) (PMMA) , polystyrene (PS), polyvinyl phloride (PVC), polyvinylalcohol-water, polyvinylalcohol (PVOH).

The film preferably comprises polyvinylalcohol (PVOH). The polyvinylalcohol may be present between 50% and 95%, preferably between 55% and 90%, more preferably between 60% and 80% by weight of the film. The polyvinylalcohol preferably comprises polyvinyl alcohol homopolymer, polyvinylalcohol copolymer preferably anionic polyvinylalcohol copolymer, or a mixture thereof. Preferably, the film comprises a polyvinylalcohol comprising or even consisting of a blend of polyvinylalcohol homopolymers and/or anionic polyvinylalcohol copolymers wherein the anionic polyvinyl alcohol copolymers preferably are selected from sulphonated and carboxylated anionic polyvinylalcohol copolymers especially carboxylated anionic polyvinylalcohol copolymers, most preferably the polyvinylalcohol comprises or even consists of a blend of two polyvinyl alcohol homopolymers or a blend of a polyvinylalcohol homopolymer and a carboxylated anionic polyvinylalcohol copolymer. Alternatively, the polyvinylalcohol comprises or even consists of a single anionic polyvinyl alcohol copolymer, most preferably a carboxylated anionic polyvinylalcohol copolymer. When the polyvinylalcohol in the film is a blend of a polyvinylalcohol homopolymer and a carboxylated anionic polyvinylalcohol copolymer, the homopolymer and the anionic copolymer preferably are present in a relative weight ratio of 90/10 to 10/90, preferably 80/20 to 20/80, more preferably 70/30 to 50/50. When the polyvinylalcohol in the film is a blend of two polyvinylalcohol homopolymers, then the first and the second polyvinylalcohol homopolymer preferably are present in a relative weight ratio of 90/10 to 10/90, preferably 80/20 to 20/80, more preferably 70/30 to 50/50. Without wishing to be bound by theory, the term "homopolymer" generally includes polymers having a single type of monomeric repeating unit (*e.g.,* a polymeric chain comprising or consisting of a single monomeric repeating unit). For the particular case of polyvinylalcohol, the term "homopolymer" further includes copolymers having a distribution of vinyl alcohol monomer units and optionally vinyl acetate monomer units, depending on the degree of hydrolysis (*e.g*., a polymeric chain comprising or consisting of vinyl alcohol and vinyl acetate monomer units). In the case of 100% hydrolysis, a polyvinylalcohol homopolymer can include only vinyl alcohol units. Without wishing to be bound by theory, the term "copolymer" generally includes polymers having two or more types of monomeric repeating units (*e.g.,* a polymeric chain comprising or consisting of two or more different monomeric repeating units, whether as random copolymers, block copolymers, etc.). For the particular case of polyvinylalcohol, the term "copolymer" (or "polyvinylalcohol copolymer") further includes copolymers having a distribution of vinyl alcohol monomer units and vinyl acetate monomer units, depending on the degree of hydrolysis, as well as at least one other type of monomeric repeating unit (*e.g.,* a ter- (or higher) polymeric chain comprising or consisting of vinyl alcohol monomer units, vinyl acetate monomer units, and one or more other monomer units, for example anionic monomer units). In the case of 100% hydrolysis, a polyvinylalcohol copolymer can include a copolymer having vinyl alcohol units and one or more other monomer units, but no vinyl acetate units. Without wishing to be bound by theory, the term "anionic copolymer" includes copolymers having an anionic monomer unit comprising an anionic moiety. General classes of anionic monomer units which can be used for the anionic polyvinyl alcohol co-polymer include the vinyl polymerization units corresponding to monocarboxylic acid vinyl monomers, their esters and anhydrides, dicarboxylic monomers having a polymerizable double bond, their esters and anhydrides, vinyl sulfonic acid monomers, and alkali metal salts of any of the foregoing. Examples of suitable anionic monomer units include the vinyl polymerization units corresponding to vinyl anionic monomers including vinyl acetic acid, maleic acid, monoalkyl maleate, dialkyl maleate, monomethyl maleate, dimethyl maleate, maleic anyhydride, fumaric acid, monoalkyl fumarate, dialkyl fumarate, monomethyl fumarate, dimethyl fumarate, fumaric anyhydride, itaconic acid, monomethyl itaconate, dimethyl itaconate, itaconic anhydride, vinyl sulfonic acid, allyl sulfonic acid, ethylene sulfonic acid, 2-acrylamido-1-methylpropanesulfonic acid, 2-acrylamido-2-methylpropanesulfonic acid, 2-methylacrylamido-2-methylpropanesulfonic acid, 2-sufoethyl acrylate, alkali metal salts of the foregoing (e.g., sodium, potassium, or other alkali metal salts), esters of the foregoing (*e.g*., methyl, ethyl, or other C1-C4 or C6 alkyl esters), and combinations thereof (*e.g*., multiple types of anionic monomers or equivalent forms of the same anionic monomer). The anionic monomer may be one or more acrylamido methylpropanesulfonic acids (*e.g*., 2-acrylamido-1-methylpropanesulfonic acid, 2-acrylamido-2-methylpropanesulfonic acid, 2-methylacrylamido-2-methylpropanesulfonic acid), alkali metal salts thereof (*e.g*., sodium salts), and combinations thereof. Preferably, the anionic moiety of the first anionic monomer unit is selected from a sulphonate, a carboxylate, or a mixture thereof, more preferably a carboxylate, most preferably an acrylate, a methacrylate, a maleate, or a mixture thereof. Preferably, the anionic monomer unit is present in the anionic polyvinyl alcohol copolymer in an average amount in a range of between 1 mol.% and 10 mol.%, preferably between 2 mol.% and 5 mol.%. Preferably, the polyvinyl alcohol, and/or in case of polyvinylalcohol blends the individual polyvinylalcohol polymers, have an average viscosity (µ1) in a range of between 4 mPa.s and 30 mPa.s, preferably between 5 mPa.s and 25 mPa.s, measured as a 4% polyvinyl alcohol copolymer solution in demineralized water at 20 degrees C. The viscosity of a polyvinyl alcohol polymer is determined by measuring a freshly made solution using a Brookfield LV type viscometer with UL adapter as described in British Standard EN ISO 15023-2:2006 Annex E Brookfield Test method. It is international practice to state the viscosity of 4% aqueous polyvinyl alcohol solutions at 20°C. It is well known in the art that the viscosity of an aqueous water-soluble polymer solution (polyvinylalcohol or otherwise) is correlated with the weight-average molecular weight of the same polymer, and often the viscosity is used as a proxy for weight-average molecular weight. Thus, the weight-average molecular weight of the polyvinylalcohol can be in a range of 20,000 to 175,000, or 25,000 to 100,000, or 30,000 to 80,000. Preferably, the polyvinyl alcohol, and/or in case of polyvinylalcohol blends the individual polyvinylalcohol polymers, have an average degree of hydrolysis in a range of between 75% and 99%, preferably between 80% and 95%, most preferably between 85% and 95%. A suitable test method to measure the degree of hydrolysis is as according to standard method JIS K6726.

Preferably, the film comprises a non-aqueous plasticizer. Preferably, the non-aqueous plasticizer is selected from polyols, sugar alcohols, and mixtures thereof. Suitable polyols include polyols selected from the group consisting of glycerol, diglycerin, ethylene glycol, diethylene glycol, triethyleneglycol, tetraethylene glycol, polyethylene glycols up to 400 molecular weight, neopentyl glycol, 1,2-propylene glycol, 1,3-propanediol, dipropylene glycol, polypropylene glycol, 2-methyl-1,3-propanediol, trimethylolpropane and polyether polyols, or a mixture thereof. Suitable sugar alcohols include sugar alcohols selected from the group consisting of isomalt, maltitol, sorbitol, xylitol, erythritol, adonitol, dulcitol, pentaerythritol and mannitol, or a mixture thereof. More preferably the non-aqueous plasticizer is selected from glycerol, 1,2-propanediol, dipropylene glycol, 2-methyl-1,3-propanediol, trimethylolpropane, triethyleneglycol, polyethyleneglycol, sorbitol, or a mixture thereof, most preferably selected from glycerol, sorbitol, trimethylolpropane, dipropylene glycol, and mixtures thereof. One particularly suitable plasticizer system includes a blend of glycerol, sorbitol and trimethylol propane. Another particularly suitable plasticizer system includes a blend of glycerin, dipropylene glycol, and sorbitol. Preferably, the film comprises between 5% and 50%, preferably between 10% and 40%, more preferably between 20% and 30% by weight of the film of the non-aqueous plasticizer.

Preferably, the film comprises a surfactant. Preferably, the film comprises a surfactant in an amount between 0.1% and 2.5%, preferably between 1% and 2% by weight of the film. Suitable surfactants can include the nonionic, cationic, anionic and zwitterionic classes. Suitable surfactants include, but are not limited to, polyoxyethylenated polyoxypropylene glycols, alcohol ethoxylates, alkylphenol ethoxylates, tertiary acetylenic glycols and alkanolamides (nonionics), polyoxyethylenated amines, quaternary ammonium salts and quaternized polyoxyethylenated amines (cationics), and amine oxides, N-alkylbetaines and sulfobetaines (zwitterionics). Other suitable surfactants include dioctyl sodium sulfosuccinate, lactylated fatty acid esters of glycerol and propylene glycol, lactylic esters of fatty acids, sodium alkyl sulfates, polysorbate 20, polysorbate 60, polysorbate 65, polysorbate 80, lecithin, acetylated fatty acid esters of glycerol and propylene glycol, and acetylated esters of fatty acids, and combinations thereof.

Preferably the film comprises one or more lubricants / release agents. Suitable lubricants/release agents can include, but are not limited to, fatty acids and their salts, fatty alcohols, fatty esters, fatty amines, fatty amine acetates and fatty amides. Preferred lubricants/release agents are fatty acids, fatty acid salts, and fatty amine acetates. The amount of lubricant/release agent in the film is in a range of from 0.02% to 1.5%, preferably from 0.1% to 1% by weight of the film.

Preferably, the film comprises fillers, extenders, antiblocking agents, detackifying agents or a mixture thereof. Suitable fillers, extenders, antiblocking agents, detackifying agents or a mixture thereof include, but are not limited to, starches, modified starches, crosslinked polyvinylpyrrolidone, crosslinked cellulose, microcrystalline cellulose, silica, metallic oxides, calcium carbonate, talc and mica. Preferred materials are starches, modified starches and silica. Preferably, the amount of filler, extender, antiblocking agent, detackifying agent or mixture thereof in the film is in a range of from 0.1% to 25%, preferably from 1% to 10%, more preferably from 2% to 8%, most preferably from 3% to 5% by weight of the film. In the absence of starch, one preferred range for a suitable filler, extender, antiblocking agent, detackifying agent or mixture thereof is from 0.1% to 1%, preferably 4%, more preferably 6%, even more preferably from 1% to 4%, most preferably from 1% to 2.5%, by weight of the film.

The film may comprise an aversive agent, for example a bittering agent. Suitable bittering agents include, but are not limited to, naringin, sucrose octaacetate, quinine hydrochloride, denatonium benzoate, or mixtures thereof. Any suitable level of aversive agent may be used in the film. Suitable levels include, but are not limited to, 1 to 5000ppm, or even 100 to 2500ppm, or even 250 to 2000rpm.

Preferably, the film or product or both are coated in a lubricating agent, preferably, wherein the lubricating agent is selected from talc, zinc oxide, silicas, siloxanes, zeolites, silicic acid, alumina, sodium sulphate, potassium sulphate, calcium carbonate, magnesium carbonate, sodium citrate, sodium tripolyphosphate, potassium citrate, potassium tripolyphosphate, calcium stearate, zinc stearate, magnesium stearate, starch, modified starches, clay, kaolin, gypsum, cyclodextrins or mixtures thereof.

Preferably, the film, and each individual component thereof, independently comprises between 0 ppm and 20 ppm, preferably between 0 ppm and 15 ppm, more preferably between 0 ppm and 10 ppm, even more preferably between 0 ppm and 5 ppm, even more preferably between 0 ppm and 1 ppm, even more preferably between 0 ppb and 100 ppb, most preferably 0 ppb dioxane. Those skilled in the art will be aware of known methods and techniques to determine the dioxane level within films and ingredients thereof.

Preferably the film according to the invention has a residual moisture content of at least 4%, more preferably in a range of from 4% to 15%, even more preferably of from 5% to 10% by weight of the film as measured by Karl Fischer titration.

Films described herein are commercially supplied by MonoSol, LLC under the trade references M8630, M8900, M8779, M8310, by Aicello Corporation under the Solublon tradename, by Nippon Gohsei under the Hi-Selon tradename, or by Sekisui Chemical under the Selvol tradename.

The film is preferably a polyvinyl alcohol (PVOH) film. Examples of water soluble PVOH films include Solublon^{®} (manufactured by Aicello), Selvol^{™} (manufactured by Sckisui), Poval^{™}( manufactured by Kuraray), Hi-Selon (manufactured by Nippon Gohsei), and PVOH films manufactured by Soltec.

The film may be opaque, transparent or translucent. The film may comprise a printed area. The area of print may be achieved using standard techniques, such as flexographic printing or inkjet printing. Preferably, the ink used in the printed area comprises between 0ppm and 20ppm, preferably between 0ppm and 15ppm, more preferably between 0ppm and 10ppm, even more preferably between 0ppm and 5ppm, even more preferably between 0ppm and 1ppm, even more preferably between 0ppb and 100ppb, most preferably 0ppb dioxane. Those skilled in the art will be aware of known methods and techniques to determine the dioxane level within the ink formulations.

The film may be incorporated into a product. The product is typically a manufactured product. The product preferably holds a substance (*e.g*. detergent, food product, pharmaceutical and agricultural chemical, usually liquid) sealed within the film by the bond. The product is preferably a soluble container or pouch containing a liquid, preferably containing a liquid detergent. The pouch is particularly used to contain a dose laundry detergent. Because the film is soluble, it enables release of liquid during washing. The pouch typically contains at least one lumen for holding the substance, where the lumen is defined by an upper film wall, a lower film wall, and edges which are bonded by wetting of the film. The present measurement of adhesability is a prediction of a bond strength in the product, in particular, in a pouch.

Aqueous liquid may be or comprise tap water, de-ionised water, or distilled water, preferably de-ionised water.

To achieve the contacting of the straight side edge (102) of the film sample (108) with the aqueous liquid (160), the film sample is disposed onto a solid support. Aqueous liquid is introduced onto the solid support such that it flows towards the straight side edge (102). Preferably, only the straight side edge (102) is contacted with the aqueous liquid (160).

Preferably, the film sample (108) is disposed suspended or clamped in a flow cell, and the aqueous liquid (160) is introduced to the straight side edge (102) under a force of gravity or by capillary action. In a preferred example, the flow cell comprises an upper layer of transparent material and a lower layer of transparent, translucent or opaque material, and the film sample is suspended between the upper layer and lower layer. The layers are preferably rigid. A longitudinal channel (*e.g*. engraved channel) is provided preferably in the upper layer on the side facing the lower layer. A first end of the channel is connected to a well (hole) in the upper layer and a second end of the channel terminates adjacent to (but without overlapping with) the straight side edge (102) of the film sample (108). Aqueous liquid (160) introduced into the well flows along the channel under gravity, and passes from the second end of the channel to the straight side edge (102) by capillary action. The upper layer of transparent material allows passage of transmitted or reflected light for capture by the microscope.

Swelling rate is determined from a (change in) position (*p1*) over time of the straight side edge (102) during the contacting, when viewed on the first surface (106). The position (*p1*) over time of the straight side edge (102) is determined relative to the position (*p0*) of the straight side edge (102) prior to contact with the aqueous liquid (160).

Penetration rate is determined from a (change in) position (*p2*) over time of the liquid front (162) into the film sample during the contacting, when viewed on the first surface (106) **(****FIGs. 2** and **3****)** . The position (*p2*) over time of the liquid front (162) is determined relative to the position (*p0*) of the straight side edge (102) prior to contact with the aqueous liquid (160).

The change in position is preferably determined with respect to a measurement axis (a-a') disposed perpendicular to the straight side edge (102).

As shown, for instance, in **FIG. 2****,** the film sample (108) straight side edge (102) is brought into contact with a liquid front (162) of the aqueous liquid (160). During contacting (*e.g*. **FIG. 3****),** the position of liquid front (162) changes (*p0 cf p2*) due to advancing into the film sample (108) and simultaneously the position of the straight side edge (102) moves (*p0 cf p1*) due to swelling of the film sample (108). A measurement axis (a-a') is indicated.

The expansion parameters may be measured optically, preferably using a light microscope. The light microscope may operate in transmission mode (Brightfield or polarized light) or in reflection mode. The microscope is disposed with an image capture device, comprising an imaging sensor for recording of images of the film sample (108). An example of a suitable light microscope is Zeiss Axiovert 200M.

Optical image is taken wherein at least a part of the first surface (106) is orientated to face the imaging device, in particular, the imaging sensor. Optical image is taken wherein the first surface (106) is within a focal plane of the microscope.

Optical image is taken wherein at least a part of the straight side edge (102) is within the field of view the microscope. Optical image is taken wherein at least a part of the straight side edge (102) is within a focal plane of the microscope.

The film sample may be disposed in any orientation with respect to the global horizon.

A plurality of optical images is captured by the image capture device during contacting the straight side edge (102) of the film sample with the aqueous liquid (160).

An image dataset comprises a plurality of optical images (200, a,b,c). Each image depicts at least a portion of the first surface (106) and of the straight side edge (102) of the film sample. Each optical image (200, a, b, c) in the image dataset is captured at a different time point during the contacting. At least one optical image of the plurality is captured prior to contacting the straight side edge (102) with the aqueous liquid (160).

**FIGs. 4A to 4C** exemplarily show a plurality of images (200, a to c) during contacting the straight side edge (102) of the film sample with the aqueous liquid (160), each image taken at a different time point (t=1 **(****FIG. 4A****),** t=2 **(****FIG. 4B****),** t=z **(****FIG. 4C****)).** Each image has at least:
- a first front edge (202, a,b,c) corresponding to a straight side edge (102) of the film sample (108), and
- a second front edge (262; a,b,c) corresponding to a liquid front (162) of the aqueous liquid (160) penetrating the film sample (108).

Each image (200, a to c) may further show
- an aqueous liquid part (260) corresponding to the aqueous liquid (160);
- a film sample part (208) corresponding to the film sample (108);
- a first surface part (206) corresponding to the first surface (106);

The position of the first front edge (202, a,b,c) prior to contact of the film sample with the aqueous liquid and the position of the second front edge (262a,b,c) prior to penetration into the film is shown by line *p0.* The change of position (*p1*) of the first front edge (222, a,b,c) and the change in position (*p2*) of the second front edge (262, a,b,c) may be determined relative to the position (*p0*) of the first front edge (222, a,b,c) prior to contact with the aqueous liquid.

The position (*p0, p1*) of the first front edge (202, a,b,c), the position (*p2*) of second front edge (262a,b,c), and may be determined for the full frame optical image (200,a,b,c), or for (only) an image portion (200',a,b,c), that is a subregion of the optical image (200,a,b,c) that contains the straight side edge (102) and liquid front (162) both before swelling has begin and until the swelling has stopped. The position and size of the image portion (200',a,b,c) remains the same for each optical image (200,a,b) in the image dataset. The image portion (200',a,b,c) is preferably rectangular (*e.g*. square or oblong) . One straight edge of image portion (200',a,b) is preferably disposed parallel to the first front edge (202, a,b,c), or disposed parallel to an average of the first front edge (202, a,b,c) in optical images (200,a,b,c) of the image dataset.

Some time after contacting, the position (*p1*) of the first front edge (202, a,b,c) changes with respect to the starting position (*p0*)*.* The change is due to swelling of the film sample. By measuring the change in *p1* over time, the swelling rate of the film sample (108) can be determined from the plurality of optical images (200, a to c) or image portions (200',a,b,c). More in particular, by measuring the change in *p1* over time relative to *p0,* the swelling rate of the film sample (108) can be determined from the plurality of optical images (200, a to c) or image portions (200',a,b,c).

Some time after contacting, the position (*p2)* of the second front edge (262a,b,c) changes with respect to the starting position (*p0*). The change is due to advancing penetration of the aqueous liquid (160) into the film sample (108). By measuring the change in *p2* over time, the penetration rate of the aqueous liquid (160) can be determined from the plurality of optical images (200, a to c) or image portions (200',a,b,c). More in particular, by measuring the change in *p2* over time relative to *p0,* the penetration rate of the aqueous liquid (160) can be determined from the plurality of optical images (200, a to c) or image portions (200',a,b,c).

The set of expansion parameters is preferably measured comprising the steps:
- generating an image dataset comprising a plurality of optical images (200, a,b,c) of at least a portion of the straight side edge (102), each optical image (200, a, b, c) captured at a different time point during the contacting; and
- identifying in each optical image (200, a, b, c) or image portion (200',a,b,c) thereof

a first front edge (202, a,b,c) corresponding to a straight side edge (102) of the film sample (108), and a second front edge (262a,b,c) corresponding to a liquid front (162) of the aqueous liquid (160) penetrating the film sample (108); and
wherein the swelling rate is determined from a change in position (*p1*) of the first front edge (202, a,b,c) over the different timepoints, and the penetration rate is determined from a change in position (*p2*) of the second front edge (262a,b,c) over the different timepoints.

Preferably, images are collected for 8 to 20 seconds, preferably 8 to 14 seconds after initial contact of straight side edge (102) with the aqueous liquid (160).

The first front edge (202, a,b,c) and second front edge (262a,b,c) may be automatically identified in each optical image (200, a,b,c) or in each image portion (200',a,b,c) by enhancing the optical image (200, a,b,c) or image portion (200',a,b,c) to emphasise the first front edge (202, a,b,c) and the second front edge (262a,b,c). Thereby, the swelling rate and penetration rate may be automatically measured. The image (200, a,b,c) so enhanced is an enhanced image (300, a,b). The image portion (200',a,b) so enhanced is an enhanced image portion (300',a,b). Examples of enhanced images (300, a,b) are shown in **FIGs. 6A** and **6B****,** which are generated from the respective optical images (200, a,b) and optical image portions (200, a,b) shown in **FIGs. 5A** and B at times t=1 and t=2 respectively. Examples of enhanced image portions (300', a,b) are shown in **FIGs. 9A** and **9B****,** which are generated from the respective optical images (200, a,b) and optical image portions (200, a,b) shown in **FIGs. 8A** and **8B** at times t=1 and t=2 respectively.

The first front edge (202, a,b) in the optical image (200, a,b) or image portion (200',a,b) corresponds to an enhanced first region (302, a,b) in the enhanced image (300, a,b) or enhanced image portion (300',a,b). The second front edge (262, a,b) in an optical image (200, a,b) image portion (200',a,b) corresponds to an enhanced second region (362,a,b) in the enhanced image (300, a,b) or the enhanced image portion (300',a,b).

Preferably, the enhanced first region (302, a,b,c) has a grey intensity that is different compared with regions flanking the enhanced first region (302, a,b,c). Preferably, the enhanced first region (302, a,b,c) has a grey intensity that is higher (lighter) compared with regions flanking the enhanced first region (302, a,b,c). Where, on a numeric scale of grey intensity, a darker shade has a lower value than a lighter shade, preferably the enhanced first region (302, a,b,c) has a grey intensity having a greater value compared with regions flanking the enhanced first region (302, a,b,c). Where, on a numeric scale, a grey intensity of 0 is black and of 255 is white, preferably the enhanced first region (302, a,b,c) has a grey intensity having a greater value compared with regions flanking the enhanced first region (302, a,b,c), preferably at least 3% to 10% greater.

Preferably, the enhanced second region (362, a,b) has a grey intensity that is different compared with regions flanking the enhanced second region (362, a,b). Preferably, the enhanced second region (362, a,b) has a grey intensity that is higher (lighter) compared with regions flanking the enhanced second region (362, a,b). Where, on a numeric scale of grey intensity, a darker shade has a lower value than a lighter shade, preferably the enhanced second region (362, a,b) has a grey intensity having a greater value compared with regions flanking the enhanced second region (362, a,b). Where on a numeric scale a grey intensity of 0 is black and of 255 is white, preferably the enhanced second region (362, a,b) has a grey intensity having a greater value compared with regions flanking the enhanced second region (362, a,b), preferably at least 3% to 10% greater.

The enhanced first region (302, a,b,c) and the enhanced second region (362, a,b,c) have the highest grey intensities compared with other regions (*e.g*. regions between them, or to either side of them). Having the highest intensities allows them to be automatically identified.

Preferably, the enhancing comprises the steps:
- for each optical image (200, a,b,c ) in the image dataset or image portion (200', a, b, c) thereof in the image dataset:
   - with the first front edge (202, a,b,c) aligned parallel to a y-axis, and an x-axis being perpendicular to the y-axis;
   - applying a first filter to the optical image (200, a,b) or image portion (200',a,b), comprising replacing each pixel of each column (C1 to C4) of pixels parallel to the y-axis into an average of grey intensity for that column, a column being parallel to the y-axis;
   - optionally applying (consecutively) one or more edge-enhancing filters to each previously-filtered image;
   - thereby obtaining an enhanced image (300, a,b) or enhanced image portion (300',a,b).

An example of an arrange of pixels of the optical image (200) or of the image portion thereof (200) is shown in **FIG. 11****,** wherein each column (C1 to C4) of pixels grey intensities is parallel to an average of direction of the first front edge (202, a,b,c). In **FIG. 12****,** the pixels in each column (C1 to C4, FIG. 11) of optical image (200) or of the image portion thereof (200) have each been replaced with a value that is an average of pixels for each column (C1' to C4').

The edge-enhancing filter may be:
- a Sobel filter applied along the x-axis (1st derivative of grey intensity); or
- a Laplace filter applied along the x-axis.

Preferably after applying the first filter, the Sobel filter is applied, followed by the Laplace filter.

The Sobel filter is known in the art. See for instance Kanopoulos, N., Vasanthavada, N., & Baker, R. L. (1988) Design of an image edge detection filter using the Sobel operator, IEEE Journal of Solid-State Circuits, 23(2), 358-367. The filter is applied along the x-axis. The kernel size depends on the size of the optical image (200, a,b,c) or in each image portion (200',a,b,c). As a guidance, values between 20 and 40 may be used. An example for software suitable for applying the Sobel filter includes Open CV (opencv.org).

The Laplace filter is known in the art. See for instance Dhar, R., Gupta, R., and Baishnab, K.L. (2014) An Analysis of CANNY and LAPLACIAN of GAUSSIAN Image Filters in Regard to Evaluating Retinal Image, DOI:10.1109/ICGCCEE.2014.6922270. The filter is applied along the x-axis. The kernel size depends on the size of the optical image (200, a,b,c) or in each image portion (200',a,b,c). As a guidance, values between 20 and 40 may be used. An example for software suitable for applying the Laplace filter includes Open CV (opencv.org).

The enhanced first region (302, a,b) and the enhanced second region (362, a,b) are bands both aligned with the y-axis.

The enhanced first region (302, a,b) corresponds to the first front edge (202, a,b,c) of the optical image (200,a,b) or image portion (200',a,b), that corresponds to the straight side edge (102) of the film sample (108). The position (*p0, p1*) of the enhanced first region (302,a, b) corresponds to the position (*p0, p1*) of the first front edge (202, a,b,c). By measuring the change in *p1* over time (relative to *p0*), the swelling rate of the film sample (108) can be determined from the plurality of images (200, a to c) or image portions (200',a,b).

The enhanced second region (362,a, b) corresponds to the second front edge (262a,b,c) of the optical image (200,a,b) or image portion (200',a,b), that corresponds to the liquid front (162) of the aqueous liquid (160) penetrating the film sample (108). The position (*p2*) of the enhanced second region (362,a, b) corresponds to the position (*p2*) of the second front edge (262, a,b,c). By measuring the change in *p2* over time (relative to *p0*), the penetration rate of the aqueous liquid can be determined from the plurality of images (200, a to c) or image portions (200',a,b).

The image portion (200',a,b) may be rectangular (*e.g*. square or oblong) and rotated relative to the corresponding optical image (200, a,b) such that one straight edge of the rectangle is parallel with the first front edge (202, a,b,c). In other words, one straight edge of the rectangle is aligned parallel with the aforementioned y-axis. The enhancing may be performed on the image portion (200',a,b).

The one straight edge of the rectangular image portion (200',a,b) may aligned parallel with the first front edge (202, a,b,c) by a protocol comprising the steps:
- for each optical image (200, a,b,c) in the image dataset:
   - applying a Gaussian smoothing filter;
   - subsequently applying a Laplace filter (highlight edges);
   - subsequently thresholding the image with Otsu's method;
   - subsequently identify the strongest edge lines using a Hough transform;
   - subsequently fitting *a* first line to the edge identified by the Hough transform;
   - subsequently generate a second line perpendicular to the first line;
- rotating rectangular image portion (200',a,b) such that one straight edge of the image portion (200') that it is parallel to an average rotation of the second line of the optical images (200, a,b,c) in the image dataset.

The Gaussian smoothing filter is known in the art. See for instance en.wikipedia.org/wiki/Gaussian_filter. As a guidance, an aperture of between 20 and 40 may be used. An example for software suitable for applying the Gaussian smoothing filter includes Open CV (opencv.org).

The Laplace filter is known in the art, as mentioned elsewhere herein. The filter is applied along the x-axis. The kernel size depends on the size of the optical image (200, a,b,c) or in each image portion (200',a,b,c). As a guidance, values between 20 and 40 may be used.

Otsu's method is known in the art. See for example, Nobuyuki Otsu, A threshold selection method from gray-level histograms (1979) IEEE Trans. Sys. Man. Cyber. 9 (1): 62-66. doi:10.1109/TSMC.1979.4310076. An example for software suitable for applying Otsu's method includes Open CV (opencv.org).

Hough transform is known in the art. See for example, U.S. Patent 3,069,654, en.wikipedia.org/wiki/Hough_transform. Purely as guidance, the following parameters were set resolution=1, angleresolution=1radian, threshold=20, minLineWidth=30% of image width, gap_between_lines=10. An example for software suitable for applying the Hough transform includes Open CV (opencv.org).

The position and rotation of the rectangular image portion (200',a,b) is the same for every optical image (200, a,b,c) in the image dataset.

The enhanced first region (302,a, b) and enhanced second region (362,a, b)) may be automatically identified from the enhanced image (300,a, b) by generating an intensity profile (400,a,b) from the enhanced image (300,a, b) or enhanced image portions (300', a,b).

The intensity profile (400,a, b) indicates along one axis (e.g. y-axis) pixel intensity of the enhanced image (302,a,b) or enhanced image portion (300', a,b) as an amplitude, and along another axis (*e.g.* x-axis) position along the x-axis of the enhanced image (302,a,b) or enhanced image portion (300', a,b). Since the pixels have the same column intensity, the x-axis of the enhanced image (302,a,b) or enhanced image portion (300', a,b) can be at any y-axis position. A first peak (402,a,b) in the intensity profile (400,a,b) corresponds to the enhanced first region (302,a, b). A second peak (462,a,b) in the intensity profile (400,a,b) corresponds to enhanced second region (362,a, b). Examples of intensity profiles (400,a,b) are shown in **FIGs. 7A** and **7B****,** which are generated from the respective enhanced image (302,a,b) shown in **FIGs. 6A** and **6B** at times t=1 and t=2 respectively. Examples of intensity profiles (400,a,b) are shown in **FIGs. 10A** and **10B****,** which are generated from the respective enhanced image portions (300', a,b) shown in **FIGs. 9A** and **9B** at times t=1 and t=2 respectively.

The first peak (402,a,b) and the second peak (462,a,b) have the highest maximum amplitudes compared with other regions (*e.g*. regions between them or to the side of them). Having the maximum amplitudes allows them to be automatically identified.

The first peak (402,a,b) in the intensity profile (400,a,b) corresponds to the enhanced first region (302,a, b), that corresponds to the first front edge (202, a,b,c), that corresponds to the straight side edge (102) of the film sample (108). The position (*p0, p1*) of the first peak (402,a,b) corresponds to the position (*p0, p1*) of the enhanced first region (302,a, b), that corresponds to the position (*p0, p1*) of the first front edge (202, a,b,c). By measuring the change in *p1* over time (relative to *p0*), the swelling rate of the film sample (108) can be determined from the plurality of images (200, a to c).

The second peak (462,a,b) in the intensity profile (400,a,b) corresponds to enhanced second region (362,a, b), that corresponds to the second front edge (262a,b,c), that corresponds to the liquid front (162) of the aqueous liquid (160) penetrating the film sample (108). The position (*p2*) of the second peak (462,a,b) corresponds to the position (*p2*) of the enhanced second region (362,a, b) that corresponds to the position (*p2*) of the second front edge (262, a,b,c). By measuring the change in *p2* (relative to *p0*) over time, the penetration rate of the aqueous liquid can be determined from the plurality of images (200, a to c).

Preferably, the first peak (402,a,b) has a maximum amplitude that is different compared with regions flanking the first peak (402,a,b). Where, on a numeric scale, a grey intensity of 0 is black and of 255 is white, preferably the first peak (402,a,b) has a maximum amplitude that has a greater value compared with regions flanking the first peak (402,a,b), preferably at least 3% to 10% greater.

Preferably, the second peak (462,a,b) has a maximum amplitude that is different compared with regions flanking the first peak (402,a,b). Where, on a numeric scale, a grey intensity of 0 is black and of 255 is white, preferably the second peak (462,a,b) has a maximum amplitude that has a greater value compared with regions flanking the second peak (462,a,b), preferably at least 3% to 10% greater.

Optionally one or more noise-reducing filters may be applied to the enhanced image (300,a, b) (or image portion (300',a, b) thereof) and/or to the intensity profile (400,a, b). The noise-reducing filter may be:
- a cut-off filter, to remove or diminish parts of the enhanced image (300,a, b) (or image portion (300',a, b) thereof) and/or of the intensity profile (400,a, b) which are below an intensity or amplitude threshold value.
- a stationary-selective-filter to remove or diminish parts of the enhanced image (300,a, b) (or image portion (300',a, b) thereof) and/or of the intensity profile (400,a, b) that do not change in position over the x-axis over a majority of the different time points.
- a lifespan-selective-filter to remove or diminish grey portions of the intensity profile (400,a, b) that are short-lived.

The cut-off filter is known in the art, and discards the edges (enhanced first region (302, a,b) or enhanced second region (362,a, b)) whose peak value are below the threshold. Alternatively, edges or peaks above the threshold are used to determine the position (p1) of the straight side edge (102) and position (p2) of the liquid front (162).

The stationary-selective-filter analyses all the enhanced images (300,a, b) (or image portions (300',a, b) thereof). (200',a,b) or intensity profiles (400,a, b) determined from the image dataset. The stationary-selective-filter analyses the position history of each single edge (enhanced first region (302, a,b) or enhanced second region (362,a, b)) or peak (in the intensity profiles (400,a, b)). A linear profile is fitted to a curve of each edge position or peak position over time. If the slope of this linear line is below a threshold indicated by the user (*e.g.* 4 pixels/sec), the edges or peaks are discarded. Edges or peaks that change position (slope is above a threshold) are used to determine the straight side edge (102) and the liquid front (162) in the .

The lifespan-selective-filter retains only those edges (within the enhanced images (300,a, b)) or peaks (within the intensity profiles (400,a, b)) that appear in a minimum number of consecutive frames. As a guidance, with a frame capture rate of 20 to 30 frames per second, edges or peaks appearing in at least 80 to 100 consecutive frames are retained.

The swelling rate is determined from the position (*p1*) of the straight side edge (102) of the film sample (108) over time during contacting with the aqueous liquid. The position (*p1*) of the straight side edge (102) may be determined relative to the position (*p0*) of straight side edge (102) prior to contact with the aqueous liquid.

The straight side edge (102) prior to and during contact with the aqueous liquid may be mutually parallel. A distance between the position (p1) of the straight side edge (102) of the film sample (108) during contact with the aqueous liquid and the position (*p0*) of the straight side edge (102) prior to contact with the aqueous liquid may be considered along an axis perpendicular to the straight side edge (102) before or during contact with the aqueous liquid.

The swelling rate may be determined from a time averaged window of the position (*p1*) of the straight side edge (102) over the different timepoints.

Swelling rate may be determined from the position (*p1*) at the different time points of the first front edge (202,a,b,c) each optical image (200, a,b,c) or image portion (200', a,b,c) in the image dataset.

The position of the first front edge (202,a,b,c) (*p1*) may be determined relative to the position (*p0*) of first front edge (202,a,b,c) prior to contact with the aqueous liquid.

The first front edge (202,a,b,c) prior to and during contact with the aqueous liquid may be mutually parallel. A distance between the position (p1) of the first front edge (202,a,b,c) during contact with the aqueous liquid and the position (*p0*) of the first front edge (202,a,b,c) prior to contact with the aqueous liquid may be considered along an axis perpendicular to the first front edge (202,a,b,c) before or during contact with the aqueous liquid.

The swelling rate may be determined from a time averaged window of the position (*p1*) of the first front edge (202,a,b,c) over the different timepoints.

Swelling rate may be determined from the position (*p1*) at the different time points of the enhanced first region (302,a, b) in the enhanced image (300,a,b) or enhanced image portion (300', a,b,c). The position of the enhanced first region (302,a, b) (*p1*) may be determined relative to the position (*p0*) of enhanced first region (302,a, b) prior to contact with the aqueous liquid.

The enhanced first region (302,a, b) prior to and during contact with the aqueous liquid are mutually parallel. A distance between the position (p1) of the enhanced first region (302,a, b) during contact with the aqueous liquid and the position (*p0*) of the enhanced first region (302,a, b) prior to contact with the aqueous liquid may be considered along an axis perpendicular to the enhanced first region (302,a, b) before or during contact with the aqueous liquid.

The swelling rate may be determined from a time averaged window of the position (*p1*) of the enhanced first region (302,a, b) over the different timepoints.

Swelling rate may be determined from the position (p1) at the different time points of the first peak (402,a,b) in the intensity profile (400,a,b). The position of the first peak (402,a,b) (*p1*) may be determined relative to the position (*p0*) of first peak (402,a,b) prior to contact with the aqueous liquid.

The swelling rate may be determined from a time averaged window of the position (*p1*) of the first peak (402,a,b) over the different timepoints.

Swelling rate may be non-scalar *e.g*. may be represented by a one or more parameters, by function, by a graph etc, as an evolution in the position (*p1*) over time. Swelling rate may be scalar and represented by a value. The swelling rate may be represented by a set of parameters, obtaining by fitting a first function, for instance, a first exponential function, to a curve corresponding to the position (*p1*) of the first front edge (222) at the different time points,

One example of the first function is an exponential curve Aₛ+Bₛ*exp(-Cₛ*t). Aₛ, Bₛ and Cₛ are variables that are adjusted until the function fits the curve. The parameter Cₛ corresponds to the swelling rate.

Another example of the function is a model based on Mao *et al.* (Mao et al «Hydration and swelling of dry polymers for wet adhesion»). The output is a more accurate estimation of the penetration depth and swelling rate.

The penetration rate is determined from the position (*p2*) of the liquid front (162) of the aqueous liquid (160) penetrating the film sample (108) over time during contacting with the aqueous liquid. The position of the liquid front (162) may be determined relative to the position (*p0*) of straight side edge (102) prior to contact with the aqueous liquid.

The liquid front (162) prior to and during contact with the aqueous liquid may be mutually parallel. A distance between the position (p2) of the liquid front (162) during contact with the aqueous liquid and the position (*p0*) of the straight side edge (102) prior to contact with the aqueous liquid may be considered along an axis (a-a') perpendicular to the straight side edge (102) before or during contact with the aqueous liquid.

The penetration rate may be determined from a time averaged window of the position (*p2*) (relative to *p0*) of the liquid front (162) over the different timepoints.

Penetration rate may be determined from the position (*p2*) at the different time points of the second front edge (262,a,b,c) each optical image (200, a,b,c) or image portion (200', a,b,c) in the image dataset. The position (*p2*) of the second front edge (262,a,b,c) may be determined relative to the position (*p0*) of first front edge (202,a,b,c) prior to contact with the aqueous liquid.

The second front edge (262,a,b,c) prior to and during contact with the aqueous liquid may be mutually parallel. A distance between the position (*p2*) of the second front edge (262,a,b,c) during contact with the aqueous liquid and the position (*p0*) of the first front edge (202,a,b,c) prior to contact with the aqueous liquid may be considered along an axis (a-a') perpendicular to the first front edge (202,a,b,c) before or during contact with the aqueous liquid.

The penetration rate may be determined from a time averaged window of the position (*p2*) (relative to *p0*) of the second front edge (262,a,b,c) over the different timepoints.

Penetration rate may be determined from the position (*p2*) at the different time points of the enhanced second region (362,a, b) in the enhanced image (300,a,b) or enhanced image portion (300',a,b). The position of the enhanced second region (362,a, b) (*p2*) may be determined relative to the position (*p0*) of enhanced first region (302,a, b) prior to contact with the aqueous liquid.

The enhanced second region (362,a, b) prior to and during contact with the aqueous liquid are mutually parallel. A distance between the position (*p2*) of the enhanced second region (362,a, b) during contact with the aqueous liquid and the position (*p0*) of the enhanced first region (302,a, b) prior to contact with the aqueous liquid may be considered along an axis (a-a') perpendicular to the enhanced first region (302,a, b) before or during contact with the aqueous liquid.

The penetration rate may be determined from a time averaged window of the position (*p2*) (relative to *p0*) of the enhanced second region (362,a, b) over the different timepoints.

Penetration rate may be determined from the position (*p2*) at the different time points of the second peak (462,a,b) in the intensity profile (400,a,b). The position of the second peak (462,a,b) (*p2*) may be determined relative to the position (*p0*) of first peak (402,a,b) prior to contact with the aqueous liquid.

The penetration rate may be determined from a time averaged window of the position (*p2*) (relative to *p0*) of the second peak (462,a,b) over the different timepoints.

Penetration rate may be non-scalar *e.g*. may be represented by a one or more parameters, by function, by a graph etc, as an evolution in the position (*p2*) over time. Penetration rate may be scalar and represented by a value. The penetration rate may be represented by a set of parameters, obtaining by fitting a second function, for instance, a second exponential function, to the position (*p2*) of the second front edge (262) at the different time points.

One example of the second function is an exponential curve Aₚ+Bₚ*exp(-Cₚ*t). Aₚ, Bₚ and Cₚ are variables that are adjusted until the function fits the curve. The parameter Cₚ corresponds to the penetration rate.

Another example of the function is a model based on Mao *et al* (Mao et al, Hydration and swelling of dry polymers for wet adhesion, Journal of the Mechanics and Physics of Solids, Volume 137, April 2020, 103863)

A film sample having an adhesability indicative of an acceptable predicted bonding strength has a higher swelling rate and penetration rate compared with a film sample having an adhesability indicative of an unacceptable predicted bonding strength.

The measured swelling rate and the measured penetration rate may be compared with known swelling rates and known penetration rates of samples having known adhesabilities. The measured swelling rate and the measured penetration rate that are the same as the known swelling rates and the known penetration rates of samples (within a certain tolerance) are predicted to have the same adhesability as the known adhesabilities within a certain tolerance. The known adhesabilities are preferably determined by experimentally measuring adhesabilities. In other words, the known adhesabilities are determined by experimentally measuring bonding strengths, in particular, experimentally measuring the bonding strength of a film in a product.

The experimentally measured adhesability (or experimentally measured bonding strength) of the film may be determined by applying a compressive mechanical force to a product containing the film. For instance, an external compressive mechanical force is applied to a liquid-filled pouch, at least to a part of the pouch containing the liquid. The force at which the bond yields is a measure of the bond strength of the film, in particular, the bond strength of the film in the product. A bond yields when the liquid breaches the bond.

An example of a suitable method for experimentally measuring adhesability or experimentally measuring bonding strength is based on a capsule integrity test described in Liquid Laundry Detergent Capsules Guidelines On CLP Implementation, Version 2.0, International Association for Soaps, Detergents and Maintenance Products, 26 November 2018. The method is conducted under conditions that are similar to the conditioning atmosphere (room temperature / approximately 50% relative humidity). The capsule described in the test is the equivalent to the product, in particular to the pouch. One capsule is subjected to an increasing compression force, between two flat plates of a surface larger than the surface area of the capsule, at a rate of 200-250 mm/min. This may be achieved with standard equipment such as *e.g.* Instron model 5566, or equivalent. During the method, the capsule is preferable placed inside a transparent plastic bag to avoid spillage or splashes. The capsule positioned between the two plates that apply the force, resting on its largest surface area. While the capsule is observed, the compression force is gradually increased either until 300N is reached, or a lower force in case the bond yields (and contents are usually release). Where the bond does not yield at 300N, the adhesability or bonding strength is indicated as "pass". Where the bond yields at 300N or below, the adhesability or bonding strength is indicated as "fail".

A predictive (machine learning ML) model may be generated. The predictive model is configured to predict adhesability using the swelling rate and the penetration rate of the film sample.

The predictive model is preferably trained using a training data set comprising swelling rate measured according to the present method, penetration rate measured according to the present method, and experimentally measured adhesability (experimentally measured bonding strength), and:
- a training input to the predictive model (in training) is measured swelling rate; measured penetration rate, and an output is predicted adhesability,
- the predicted adhesability is compared with the experimentally measured adhesability,
- the predictive model (in training) is adjusted so that the outputted predicted adhesability approaches the experimentally measured adhesability of the training set.

The experimentally determined adhesability is preferably an indication of "pass" or "fail".

Examples of suitable predicting model and training protocols include linear regression, random forest and neural networks which are know in the art.

Preferably, the determining from the expansion parameters, the adhesability of the film sample (108), comprises the steps:
- receiving as an input to a trained predictive model the set of expansion parameters,
- outputting from the trained predictive model the adhesability of the film sample (108).

The adhesability is preferably an indication of "pass" or "fail".

Provided herein is a computer-implemented method for determining an adhesability of a film sample (108) using a method as described herein.

An unclaimed example useful to understand the present invention is a system comprising a processor adapted to perform the computer-implemented method.

An unclaimed example useful to understand the present invention is a computer program [product] comprising instructions which, when the program is executed by a computer, cause the computer to carry out the computer-implemented method.

An unclaimed example useful to understand the present invention is a computer-readable [storage] medium comprising instructions which, when executed by a computer, cause the computer to carry out [the steps of] the computer-implemented method.

An unclaimed example useful to understand the present invention is a system comprising:
- a processor adapted to perform the computer-implemented method, and
- a light microscope disposed with an image capture device configured for acquisition of the image dataset.

In a preferred embodiment the method according to the invention is a computer-implemented method for determining an adhesability of a film sample (108), wherein adhesability is a prediction of bonding strength between the film sample and a substrate, wherein the film sample (108) is a sample of a wet-adhesion film, the method comprising:
- receiving an image dataset comprising a plurality of optical images (200, a,b,c) of at least a portion of a straight side edge (102) of the film sample (108), each optical image (200, a,b,c) captured at a different time point during contacting the straight side edge (102) of the film sample (108) with an aqueous liquid (160),
- identifying in each optical image (200, a,b,c) or an image portion (200', a,b,c) thereof, a first front edge (202, a,b,c) corresponding to the straight side edge (102), and a second front edge (262a,b,c) corresponding to a liquid front (162) of the aqueous liquid (160) penetrating the film sample (108); and
- determining a set of expansion parameters comprising:
   - a swelling rate of the film sample (108), and
   - a penetration rate of the aqueous liquid (160) into the film sample (108),
   wherein the swelling rate is determined from a change in position (*p1*) of the first front edge (202, a,b,c) over the different timepoints, and the penetration rate is determined from a change in position (*p2*) of the second front edge (262a,b,c) over the different timepoints,
- determining from the expansion parameters, the adhesability of the film sample (108).

Any of the aforementioned features described herein may be applied to the computer-implemented method.

An unclaimed example useful to understand the present invention is a system comprising a processor adapted to perform the computer-implemented method. The processor may be comprised in a remote server (cloud).

An unclaimed example useful to understand the present invention is a system comprising:
- a processor adapted to perform the computer-implemented method. The processor may be comprised in a remote server (cloud), and
- a light microscope disposed with an image capture device configured for acquisition of the image dataset.

The system may further comprise a solid support to support the film sample. The system may further comprise a flow cell configured to clamp the film sample.

An unclaimed example useful to understand the present invention is a computer program [product] comprising instructions which, when the program is executed by a computer, cause the computer to carry out the computer-implemented method.

An unclaimed example useful to understand the present invention is a computer-readable [storage] medium comprising instructions which, when executed by a computer, cause the computer to carry out [the steps of] the computer-implemented method.

The computer implemented method may output the result in a report.

In a preferred embodiment the method according to the invention is a computer-implemented method for determining an adhesability of a film sample (108), wherein adhesability is a prediction of bonding strength between the film sample and a substrate, wherein the film sample (108) is a sample of a wet-adhesion film, the method comprising:
- sending, to a remote processor, an image dataset comprising a plurality of optical images (200, a,b,c) of at least a portion of a straight side edge (102) of the film sample (108), each optical image (200, a,b,c) captured at a different time point during contacting the straight side edge (102) of the film sample (108) with an aqueous liquid (160);
- receiving from the remote processor, the adhesability of the film sample, wherein the adhesability of the film sample has been determined by:
   - identifying in each optical image (200, a,b,c) a first front edge (202, a,b,c) corresponding to the straight side edge (102), and a second front edge (262a,b,c) corresponding to a liquid front (162) of the aqueous liquid (160) penetrating the film sample (108); and
   - determining a set of expansion parameters comprising:
      - a swelling rate of the film sample (108), and
      - a penetration rate of the aqueous liquid (160) into the film sample (108),
      wherein the swelling rate is determined from a change in position (*p1*) of the first front edge (202, a,b,c) over the different timepoints, and the penetration rate is determined from a change in position (*p2*) of the second front edge (262a,b,c) over the different timepoints,
   - determining from the expansion parameters, the adhesability of the film sample (108).

An unclaimed example useful to understand the present invention is a system comprising a processor adapted to perform the computer implemented method. A part of the processor may be comprised in a remote server (cloud).

The system may further comprise:
- a light microscope disposed with an image capture device configured for acquisition of the image dataset.

An unclaimed example useful to understand the present invention is a computer program [product] comprising instructions which, when the program is executed by a computer, cause the computer to carry out [the steps of] the computer implemented method.

An unclaimed example useful to understand the present invention is a computer-readable [storage] medium comprising instructions which, when executed by a computer, cause the computer to carry out [the steps of] the computer implemented method.

The computer implemented method may output the result in a report.

The computer implemented method, system and computer program allow the determination to be performed remotely, based on an image dataset. A local user may perform the contacting of the straight side edge (102) of the film sample with the aqueous liquid (160), and capturing of optical images. A central (*e.g*. cloud-based) server may receive the dataset and determine the adhesability of the film sample (108) from the image dataset. A central server allows for the method to be performed on local devices not having adequate image processing capability, and avoid the need for locally updating software to account for changes in the determining steps. It allows for remote prediction of bonding strength at a location of manufacture of the film that is remote from location of product manufacture; the quality of the film can be assessed prior to shipping to the location of product manufacture, and prior to usage in the product manufacturing.

The method may be performed using a standard computer system such as an Intel Architecture IA-32 based computer system 2, and implemented as programming instructions of one or more software modules stored on non-volatile (e.g., hard disk or solid-state drive) storage associated with the corresponding computer system. However, it will be apparent that at least some of the steps of any of the described processes could alternatively be implemented, either in part or in its entirety, as one or more dedicated hardware components, such as gate configuration data for one or more field programmable gate arrays (FPGAs), or as application-specific integrated circuits (ASICs), for example.

The method or system may produce an output that is:
- displayed on a screen, or
- saved to a file.

### Example

Sample of film was cut with a guillotine to form a straight side edge. The film sample was mounted within a flow cell as described herein and aqueous liquid applied to the straight side edge. A plurality of optical images was recorded, at least one optical image before contacting of the film sample with the aqueous liquid. Images were taken for a standard duration of 15secs. The plurality of image were stored in an image dataset.

**FIG. 13** is a microscope optical image (200,d) of the film sample (208) and aqueous liquid (260) at time t=0, *i.e.* just prior to contact. The straight side edge (102,d), line (*p0*)*,* and a rectangular image portion (200',d) are marked. **FIG. 14** is a microscope optical image (200,f) of the same film sample (208) and aqueous liquid (260) after contacting for 12 seconds (t=12 s). The straight side edge (102,f) corresponding line (*p0*), liquid front (262,f), and rectangular image portion (200',f) are marked.

The rectangular image portion was determined for each image in the dataset. The position and rotation of the rectangular image portion was the same for each image. The position and rotation were determined by applying, for each image in the dataset, a Gaussian smoothing filter, subsequently a Laplace filter (highlight edges), subsequently thresholding the image with Otsu method and subsequently identifying the strongest edge line using a Hough transform. A first line was fit to this line. One y-axis straight edge of the rectangular image portion was disposed parallel to an average rotation of the first line across all the images in the dataset. The position was determined such that rectangular image portion contained both the straight side edge and liquid front both before swelling had begum and until the swelling has stopped. **FIG. 15** shows one of the optical images (200,e) of the film sample (208) in the dataset taken at t=0s *i.e.* just prior to contacting, with the determined image portion (200',e) indicated.

Each rectangular image portion, having a y-axis and an x-axis perpendicular thereto was enhanced to form an enhanced portion by applying a first filter to the image portion. The first filter comprised replacing each pixel of each column of pixels parallel to the image portion y-axis into an average of grey intensity for that column, subsequently applying a Sobel filter along the x-axis, subsequently applying a Laplace filter along the x-axis.

**FIG. 16** (t=0s) shows the rectangular image portion (200',e) of FIG. 15, and the enhanced portion (300',e) generated from the image portion (200',e), after averaging each pixel column (310,e) and applying edge-detection filters (Sobel filter along the x-axis (result in 312,e), Laplace filter along the X axis (result in 300',e)) resulting in the enhanced image portion (300',e).

**FIG. 17** (t=5s) shows the rectangular image portion (200',f) generated from an image in the dataset captured five seconds after the image of **FIG. 15 / FIG. 16****,** and the enhanced portion (300',f) generated from the image portion (200',f), after averaging each pixel column (310,f) and applying edge-detection filters (Sobel filter along the x-axis (result in 312,f), Laplace filter along the X axis (result in 300',f)) resulting in the enhanced image portion (300',e).

An intensity profile was generated from each enhanced image portion. To generate the intensity profile, a plot was created wherein the y-axis corresponded to pixel intensity and x-axis corresponded to position along the x-axis of the enhanced image portion. Noise reducing filters were applied: one filter being a cut-off filter, to remove or diminish portions of the intensity profile below a threshold value, another filter being a stationary-selective-filter to remove or diminish portions of the intensity profile that do not change in position over a majority of the different time points, and a lifespan-selective-filter to remove or diminish of portions of intensity profile that are short-lived. **FIG. 16** shows the intensity profile (400,e) generated from the enhanced portion (300',e) at t=0s. **FIG. 17** shows the intensity profile (400,f) generated from the enhanced portion (300',f) some seconds later (t=5s).

The resulting intensity profile contained two main peaks of intensity (highest amplitude), the first peak corresponded to the position (*p0, p1*) of the straight side edge, and the second peak corresponded to the position (*p2*) of the liquid front. From the intensity profiles, a data set of position parameters was obtained corresponding to *p1* and *p2* relative to *p0* across the time points.

**FIG. 18** shows a plot of positions (*p0, p1*) of the straight side edge, and of positions (*p2*) of the liquid front as a function of time

A mathematic function was fitted to each of the curves of *p1* (relative to *p0*) and *p2* (relative to *p0*) over time. The mathematic function was the exponential curve A+B*exp(-C*t). The value of C for the curve of *p1* corresponds to the swelling rate, and the value of C for the curve of *p2* corresponds to the penetration rate.

By comparing the swelling rate and the penetration rate with standard ranges, the adhesability and hence predicted bonding strength of the film was determined.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A method for determining an adhesability of a film sample (108), wherein adhesability is a prediction of bonding strength between the film sample and a substrate, wherein the film sample (108) is a sample of a wet-adhesion film, said film sample (108) having a first surface (106), an opposing second surface (104) and a straight side edge (102) connecting the first surface (106) and the second surface (104), **characterised by** the following steps:
- contacting the straight side edge (102) of the film sample with an aqueous liquid (160),
- measuring, from the film sample (108), a set of expansion parameters comprising:
- a swelling rate of the film sample (108), and
- a penetration rate of the aqueous liquid (160) into the film sample (108) resulting from the contacting,
- determining from the expansion parameters, the adhesability of the film sample (108).

2. The method according to claim 1, wherein:
- measuring the set of expansion parameters comprises the following steps:
- generating an image dataset comprising a plurality of optical images (200, a,b,c) of at least a portion of the straight side edge (102), each optical image (200, a, b) captured at a different time point during the contacting;
- identifying in each optical image (200, a,b,c) or from an image portion thereof (200', a,b,c) a first front edge (202, a,b,c) corresponding to the straight side edge (102), and a second front edge (262, a,b,c) corresponding to a front edge (162) of the aqueous liquid (160) penetrating the film sample (108); and
- the swelling rate is determined from a position (*p1*) of the first front edge (202, a,b,c) over the different timepoints, and the penetration rate is determined from a position (*p2*) of the second front edge (262a,b,c) over the different timepoints.

3. The method according to claim 2, wherein identifying the first (202, a,b) and second (262, a,b) front-edges comprises the steps:
- for each optical image (200, a,b) in the image dataset, or image portion thereof (200', a,b):
- with the first front edge (202, a,b,c) aligned parallel to a y-axis, and an x-axis being perpendicular to the y-axis;
- applying a first filter to the optical image (200, a,b) or image portion (200',a,b), comprising replacing (C1' to C4') each pixel of each column (C1 to C4) of pixels parallel to the y-axis into an average of grey intensity for that column;
- optionally applying one or more edge-enhancing filters to each previously-filtered image;
- thereby obtaining an enhanced image (300, a,b) or enhanced image portion (300',a,b),
- identifying from the enhanced image (300, a,b) or enhanced image portion (300',a,b) thereof, an enhanced first region (302, a,b) and enhanced second region (362,a,b), the enhanced first (302, a,b) and second (362, a,b,c) regions have the highest grey intensities compared with other regions, wherein the enhanced first region (302, a,b) corresponds to the first front edge (202, a,b) and the enhanced second region (362,a,b) corresponds to the second front edge (262, a,b).

4. The method according to claim 3, wherein the identifying from enhanced image (300, a,b) the first front edge (222,a, b) and second front edge (262,a, b)) further comprises the steps:
- for each enhanced image (300, a,b) or enhanced image portion (300', a,b)
- determining an intensity profile (400,a, b) along the x-axis of the enhanced image (302,a,b), wherein intensity profile (400,a, b) indicates along one axis pixel intensity of the enhanced image (302,a,b) or enhanced image portion (300', a,b) as an amplitude, and along another axis position along the x-axis of the enhanced image (302,a,b) or enhanced image portion (300', a,b),
- optionally applying one or more noise-reducing filters to the intensity profile (400,a, b)
- identifying in the intensity profile (400a, b), optionally noise-filtered, a first peak (402,a,b) and a second peak (462,a,b) have the highest maximum amplitudes compared with other regions, wherein the first peak (402,a,b) corresponds to the first front edge (202, a,b) and the second peak (462,a,b) corresponds to the second front edge (262a,b,c).

5. The method according to any one of the previous claims, wherein the adhesability is determined from the swelling rate and from the penetration rate, wherein a film sample having a higher adhesability has a higher swelling rate and penetration rate compared with a film sample having a lower adhesability.

6. The method according to any one of the previous claims, wherein the determining from the expansion parameters, the adhesability of the film sample (108) comprises the steps:
- receiving as an input to a trained predictive model the set of expansion parameters,
- outputting from the trained predictive model the adhesability of the film sample (108), wherein the model has been trained using a training data set comprising swelling rate, penetration rate, and experimentally measured adhesability, and
- a training input to the predictive model is the swelling rate, the penetration rate, and a training output is predicted adhesability,
- the predicted adhesability during training is compared with the experimentally measured adhesability, and
- the predictive model is adjusted during training so that the outputted predicted adhesability approaches the experimentally measured adhesability of the training data set.

7. The method according to any one of the previous claims, wherein the swelling rate and the penetration rate are compared with known swelling rates and known penetration rates of samples having known adhesabilities, thereby determining adhesability of the film sample (108).

8. The method according to any one of the previous claims, wherein
- the swelling rate is represented as a first exponential function fitted to the position (*p1*) of the first front edge (202, a,b,c) over the different timepoints,
- the penetration rate is represented as a second exponential function fitted to the position (*p2*) of the second front edge (262, a,b,c) over the different timepoints,
wherein the first exponential function and the second exponential function are compared with known first and second exponential functions of samples having known adhesabilities, thereby determining adhesability of the film sample (108).

9. The method according to any one of the previous claims, wherein the film is a wet adhesion film containing one or more of acrylonitrile butadiene styrene, polybutadiene, polycarbonates, polysulfones, polyethylene terephthalate, poly(methyl methacrylate) , polystyrene, polyvinyl chloride, polyvinylalcohol-water, polyvinylalcohol, preferably containing polyvinyl alcohol.

10. The method according to any one of the previous claims, wherein the method is computer-implemented, and wherein measuring the set of expansion parameters comprises:
- receiving an image dataset comprising a plurality of optical images (200, a,b,c) of at least a portion of a straight side edge (102) of the film sample (108), each optical image (200, a,b,c) captured at a different time point during contacting the straight side edge (102) of the film sample (108) with an aqueous liquid (160),
- identifying in each optical image (200, a,b,c) or an image portion (200', a,b,c) thereof a first front edge (202, a,b,c) corresponding to the straight side edge (102), and a second front edge (262a,b,c) corresponding to a liquid front (162) of the aqueous liquid (160) penetrating the film sample (108); and
wherein the swelling rate is determined from a change in position (*p1*) of the first front edge (202, a,b,c) over the different timepoints, and the penetration rate is determined from a change in position (*p2*) of the second front edge (262a,b,c) over the different timepoints,
- determining from the expansion parameters, the adhesability of the film sample (108).

11. The method according to any of claims 1 to 9, wherein the method is computer-implemented, and wherein measuring the set of expansion parameters comprises:
- sending, to a remote processor, an image dataset comprising a plurality of optical images (200, a,b,c) of at least a portion of a straight side edge (102) of the film sample (108), each optical image (200, a,b,c) captured at a different time point during contacting the straight side edge (102) of the film sample (108) with an aqueous liquid (160);
- receiving from the remote processor, the adhesability of the film sample, wherein the adhesability of the film sample has been determined by:
- identifying in each optical image (200, a,b,c) a first front edge (202, a,b,c) corresponding to the straight side edge (102), and a second front edge (262a,b,c) corresponding to a liquid front (162) of the aqueous liquid (160) penetrating the film sample (108); and
wherein the swelling rate is determined from a change in position (*p1*) of the first front edge (202, a,b,c) over the different timepoints, and the penetration rate is determined from a change in position (*p2*) of the second front edge (262a,b,c) over the different timepoints,
- determining from the expansion parameters, the adhesability of the film sample (108).

## Patentansprüche

1. Verfahren zum Bestimmen einer Adhäsionsfähigkeit einer Folienprobe (108), wobei die Adhäsionsfähigkeit eine Vorhersage einer Bindungsfestigkeit zwischen der Folienprobe und einem Substrat ist, wobei die Folienprobe (108) eine Probe einer Nassadhäsionsfolie ist, wobei die Folienprobe (108) eine erste Oberfläche (106), eine gegenüberliegende zweite Oberfläche (104) und eine gerade Seitenkante (102), die die erste Oberfläche (106) und die zweite Oberfläche (104) verbindet, aufweist, **gekennzeichnet durch** die folgenden Schritte:
- Berühren der geraden Seitenkante (102) der Folienprobe mit einer wässrigen Flüssigkeit (160),
- Messen, aus der Folienprobe (108), eines Satzes von Expansionsparametern, umfassend:
- eine Quellungsrate der Folienprobe (108), und
- eine Penetrationsrate der wässrigen Flüssigkeit (160) in die Folienprobe (108), die aus dem Berühren resultiert,
- Bestimmen der Adhäsionsfähigkeit der Folienprobe (108) aus den Expansionsparametern.

2. Verfahren nach Anspruch 1, wobei:
- das Messen des Satzes von Expansionsparametern die folgenden Schritte umfasst:
- Erzeugen eines Bilddatensatzes, umfassend eine Vielzahl von optischen Bildern (200, a,b,c) von wenigstens einem Teil der geraden Seitenkante (102), wobei jedes optische Bild (200, a, b) zu einem unterschiedlichen Zeitpunkt während des Berührens aufgenommen wird;
- Identifizieren in jedem optischen Bild (200, a,b,c) oder aus einem Bildabschnitt davon (200', a,b,c) einer ersten Vorderkante (202, a,b,c), die der geraden Seitenkante (102) entspricht, und einer zweiten Vorderkante (262, a,b,c), die einer Vorderkante (162) der wässrigen Flüssigkeit (160) entspricht, die die Folienprobe (108) penetriert; und
- die Quellungsrate aus einer Position (*p1*) der ersten Vorderkante (202, a,b,c) über die unterschiedlichen Zeitpunkte bestimmt wird, und die Penetrationsrate aus einer Position (*p2*) der zweiten Vorderkante (262a,b,c) über die unterschiedlichen Zeitpunkte bestimmt wird.

3. Verfahren nach Anspruch 2, wobei das Identifizieren der ersten (202, a,b) und der zweiten (262, a,b) Vorderkante die Schritte umfasst:
- für jedes optische Bild (200, a,b) in dem Bilddatensatz oder Bildabschnitt davon (200', a,b):
- wobei die erste Vorderkante (202, a,b,c) parallel zu einer y-Achse ausgerichtet ist und eine x-Achse senkrecht zu der y-Achse ist;
- Anwenden eines ersten Filters auf das optische Bild (200, a,b) oder den Bildabschnitt (200',a,b), umfassend ein Ersetzen (C1' bis C4') jedes Pixels jeder Spalte (C1 bis C4) von Pixeln parallel zu der y-Achse durch einen Durchschnitt einer Grauintensität für diese Spalte;
- wahlweise Anwenden eines oder mehrerer kantenverbessernder Filter auf jedes zuvor gefilterte Bild;
- wodurch ein verbessertes Bild (300, a,b) oder ein verbesserter Bildabschnitt (300',a,b) erhalten wird,
- Identifizieren aus dem verbesserten Bild (300, a,b) oder dem verbesserten Bildabschnitt (300',a,b) davon, einer verbesserten ersten Region (302, a,b) und verbesserten zweiten Region (362,a,b), wobei die verbesserte erste (302, a,b) und zweite (362, a,b,c) Region die höchsten Grauintensitäten im Vergleich zu anderen Regionen aufweisen, wobei die verbesserte erste Region (302, a,b) der ersten Vorderkante (202, a,b) entspricht und die verbesserte zweite Region (362,a,b) der zweiten Vorderkante (262, a,b) entspricht.

4. Verfahren nach Anspruch 3, wobei das Identifizieren aus dem verbesserten Bild (300, a,b) der ersten Vorderkante (222,a, b) und der zweiten Vorderkante (262,a, b)) ferner die Schritte umfasst:
- für jedes verbesserte Bild (300, a,b) oder jeden verbesserten Bildabschnitt (300', a,b)
- Bestimmen eines Intensitätsprofils (400,a, b) entlang der x-Achse des verbesserten Bildes (302,a,b),
wobei das Intensitätsprofil (400,a, b) entlang einer Achse die Pixelintensität des verbesserten Bildes (302,a,b) oder verbesserten Bildabschnitts (300', a,b) als eine Amplitude und entlang einer anderen Achse die Position entlang der x-Achse des verbesserten Bildes (302,a,b) oder verbesserten Bildabschnitts (300', a,b) angibt,
- wahlweise Anwenden eines oder mehrerer rauschreduzierender Filter auf das Intensitätsprofil (400,a, b)
- Identifizieren in dem Intensitätsprofil (400a, b), wahlweise rauschgefiltert, eines ersten Peaks (402,a,b) und eines zweiten Peaks (462,a,b), die die höchsten maximalen Amplituden im Vergleich zu anderen Regionen aufweisen, wobei der erste Peak (402,a,b) der ersten Vorderkante (202, a,b) entspricht und der zweite Peak (462,a,b) der zweiten Vorderkante (262a,b,c) entspricht.

5. Verfahren nach einem der vorstehenden Ansprüche, wobei die Adhäsionsfähigkeit aus der Quellungsrate und aus der Penetrationsrate bestimmt wird, wobei eine Folienprobe, die eine höhere Adhäsionsfähigkeit aufweist, eine höhere Quellungsrate und Penetrationsrate im Vergleich zu einer Folienprobe, die eine niedrigere Adhäsionsfähigkeit aufweist, aufweist.

6. Verfahren nach einem der vorstehenden Ansprüche, wobei das Bestimmen der Adhäsionsfähigkeit der Folienprobe (108) aus den Expansionsparametern die Schritte umfasst:
- Empfangen des Satzes von Expansionsparametern als eine Eingabe für ein trainiertes Vorhersagemodell,
- Ausgeben der Adhäsionsfähigkeit der Folienprobe (108) aus dem trainierten Vorhersagemodell,
wobei das Modell unter Verwendung eines Trainingsdatensatzes trainiert wurde, umfassend Quellungsrate, Penetrationsrate und experimentell gemessene Adhäsionsfähigkeit, und
- eine Trainingseingabe für das Vorhersagemodell die Quellungsrate, die Penetrationsrate ist, und eine Trainingsausgabe die vorhergesagte Adhäsionsfähigkeit ist,
- die vorhergesagte Adhäsionsfähigkeit während des Trainings mit der experimentell gemessenen Adhäsionsfähigkeit verglichen wird, und
- das Vorhersagemodell während des Trainings so eingestellt wird, dass die ausgegebene vorhergesagte Adhäsionsfähigkeit sich der experimentell gemessenen Adhäsionsfähigkeit des Trainingsdatensatzes annähert.

7. Verfahren nach einem der vorstehenden Ansprüche, wobei die Quellungsrate und die Penetrationsrate mit bekannten Quellungsraten und bekannten Penetrationsraten von Proben, die bekannte Adhäsionsfähigkeiten aufweisen, verglichen werden, wodurch die Adhäsionsfähigkeit der Folienprobe (108) bestimmt wird.

8. Verfahren nach einem der vorstehenden Ansprüche, wobei
- die Quellungsrate als eine erste Exponentialfunktion dargestellt wird, die an die Position (*p1*) der ersten Vorderkante (202, a,b,c) über die unterschiedlichen Zeitpunkte angepasst ist,
- die Penetrationsrate als eine zweite Exponentialfunktion dargestellt wird, die an die Position (*p2*) der zweiten Vorderkante (262, a,b,c) über die unterschiedlichen Zeitpunkte angepasst ist,
wobei die erste Exponentialfunktion und die zweite Exponentialfunktion mit bekannten ersten und zweiten Exponentialfunktionen von Proben, die bekannte Adhäsionsfähigkeiten aufweisen, verglichen werden, wodurch die Adhäsionsfähigkeit der Folienprobe (108) bestimmt wird.

9. Verfahren nach einem der vorstehenden Ansprüche, wobei die Folie eine Nassadhäsionsfolie ist, die eines oder mehrere von Acrylnitrilbutadienstyrol, Polybutadien, Polycarbonaten, Polysulfonen, Polyethylenterephthalat, Poly(methylmethacrylat), Polystyrol, Polyvinylchlorid, Polyvinylalkohol-Wasser, Polyvinylalkohol enthält, vorzugsweise Polyvinylalkohol enthält.

10. Verfahren nach einem der vorstehenden Ansprüche, wobei das Verfahren computerimplementiert ist, und wobei das Messen des Satzes von Expansionsparametern umfasst:
- Empfangen eines Bilddatensatzes, umfassend eine Vielzahl von optischen Bildern (200, a,b,c) von wenigstens einem Teil einer geraden Seitenkante (102) der Folienprobe (108), wobei jedes optische Bild (200, a,b,c) zu einem unterschiedlichen Zeitpunkt während des Berührens der geraden Seitenkante (102) der Folienprobe (108) mit einer wässrigen Flüssigkeit (160) aufgenommen wird,
- Identifizieren in jedem optischen Bild (200, a,b,c) oder einem Bildabschnitt (200', a,b,c) davon einer ersten Vorderkante (202, a,b,c), die der geraden Seitenkante (102) entspricht, und einer zweiten Vorderkante (262a,b,c), die einer Flüssigkeitsfront (162) der wässrigen Flüssigkeit (160) entspricht, die die Folienprobe (108) penetriert; und
wobei die Quellungsrate aus einer Positionsänderung (*p1*) der ersten Vorderkante (202, a,b,c) über die unterschiedlichen Zeitpunkte bestimmt wird, und die Penetrationsrate aus einer Positionsänderung (*p2*) der zweiten Vorderkante (262a,b,c) über die unterschiedlichen Zeitpunkte bestimmt wird,
- Bestimmen der Adhäsionsfähigkeit der Folienprobe (108) aus den Expansionsparametern.

11. Verfahren nach einem der Ansprüche 1 bis 9, wobei das Verfahren computerimplementiert ist, und wobei das Messen des Satzes von Expansionsparametern umfasst:
- Senden, an einen entfernten Prozessor, eines Bilddatensatzes, umfassend eine Vielzahl von optischen Bildern (200, a,b,c) von wenigstens einem Teil einer geraden Seitenkante (102) der Folienprobe (108), wobei jedes optische Bild (200, a,b,c) zu einem unterschiedlichen Zeitpunkt während des Berührens der geraden Seitenkante (102) der Folienprobe (108) mit einer wässrigen Flüssigkeit (160) aufgenommen wird;
- Empfangen, von dem entfernten Prozessor, der Adhäsionsfähigkeit der Filmprobe, wobei die Adhäsionsfähigkeit der Folienprobe bestimmt wurde durch:
- Identifizieren in jedem optischen Bild (200, a,b,c) einer ersten Vorderkante (202, a,b,c), die der geraden Seitenkante (102) entspricht, und einer zweiten Vorderkante (262a,b,c), die einer Flüssigkeitsfront (162) der wässrigen Flüssigkeit (160) entspricht, die die Folienprobe (108) penetriert; und
wobei die Quellungsrate aus einer Positionsänderung (*p1*) der ersten Vorderkante (202, a,b,c) über die unterschiedlichen Zeitpunkte bestimmt wird, und die Penetrationsrate aus einer Positionsänderung (*p2*) der zweiten Vorderkante (262a,b,c) über die unterschiedlichen Zeitpunkte bestimmt wird,
- Bestimmen der Adhäsionsfähigkeit der Folienprobe (108) aus den Expansionsparametern.

## Revendications

1. Procédé permettant de déterminer une adhésivité d'un échantillon de film (108), dans lequel l'adhésivité est une prédiction de la force de collage entre l'échantillon de film et un substrat, dans lequel l'échantillon de film (108) est un échantillon d'un film d'adhérence humide, ledit échantillon de film (108) présentant une première surface (106), une seconde surface opposée (104) et un bord latéral droit (102) reliant la première surface (106) et la seconde surface (104), **caractérisé par** les étapes suivantes :
- la mise en contact du bord latéral droit (102) de l'échantillon de film avec un liquide aqueux (160),
- la mesure, à partir de l'échantillon de film (108), d'un ensemble de paramètres d'expansion comprenant :
- un taux de gonflement de l'échantillon de film (108), et
- un taux de pénétration du liquide aqueux (160) dans l'échantillon de film (108) résultant de la mise en contact,
- la détermination à partir des paramètres d'expansion, de l'adhésivité de l'échantillon de film (108).

2. Procédé selon la revendication 1, dans lequel :
- la mesure de l'ensemble des paramètres d'expansion comprend les étapes suivantes :
- la génération d'un ensemble de données d'images comprenant une pluralité d'images optiques (200, a, b, c) d'au moins une partie du bord latéral droit (102), chaque image optique (200, a, b) étant capturée à un point temporel différent pendant la mise en contact ;
- l'identification dans chaque image optique (200, a, b, c) ou à partir d'une partie d'image de celle-ci (200', a, b, c), d'un premier bord avant (202, a, b, c) correspondant au bord latéral droit (102), et un second bord avant (262, a, b, c) correspondant à un bord avant (162) du liquide aqueux (160) pénétrant dans l'échantillon de film (108) ; et
- le taux de gonflement est déterminé à partir d'une position (*p1*) du premier bord avant (202, a, b, c) sur les différents points temporels, et le taux de pénétration est déterminé à partir d'une position (*p2*) du second bord avant (262a, b, c) sur les différents points temporels.

3. Procédé selon la revendication 2, dans lequel l'identification des premier (202, a, b) et second (262, a, b) bords avant comprend les étapes :
- pour chaque image optique (200, a, b) dans l'ensemble de données d'image, ou une partie d'image de celui-ci (200', a, b) :
- avec le premier bord avant (202, a, b, c) aligné parallèlement à un axe y, et un axe x étant perpendiculaire à l'axe y ;
- l'application d'un premier filtre à l'image optique (200, a, b) ou à la partie d'image (200', a, b), comprenant le remplacement (C1' à C4') de chaque pixel de chaque colonne (C1 à C4) de pixels parallèles à l'axe y par une moyenne d'intensité de gris pour cette colonne ;
- l'application éventuelle d'un ou de plusieurs filtres d'amélioration des bords à chaque image précédemment filtrée ;
- l'obtention ainsi d'une image améliorée (300, a, b) ou d'une partie d'image améliorée (300', a, b),
- l'identification à partir de l'image améliorée (300, a, b) ou de la partie d'image améliorée (300', a, b) de celle-ci, d'une première région améliorée (302, a, b) et d'une seconde région améliorée (362, a, b), les première (302, a, b) et seconde régions améliorées (362, a, b, c) présentent les intensités de gris les plus élevées par rapport aux autres régions, dans lequel la première région améliorée (302, a, b) correspond au premier bord avant (202, a, b) et la seconde région améliorée (362, a, b) correspond au second bord avant (262, a, b).

4. Procédé selon la revendication 3, dans lequel l'identification, à partir de l'image améliorée (300, a, b), du premier bord avant (222,a, b) et du second bord avant (262,a, b) comprend en outre les étapes consistant à :
- pour chaque image améliorée (300, a, b) ou partie d'image améliorée (300', a, b)
- déterminer un profil d'intensité (400,a, b) le long de l'axe x de l'image améliorée (302,a,b),
dans lequel le profil d'intensité (400,a, b) indique le long d'un axe l'intensité de pixel de l'image améliorée (302,a,b) ou de la partie d'image améliorée (300', a,b) comme amplitude, et le long d'une autre position d'axe le long de l'axe x de l'image améliorée (302,a,b) ou de la partie d'image améliorée (300', a,b),
- appliquer éventuellement un ou plusieurs filtres antibruit au profil d'intensité (400,a, b)
- identifier dans le profil d'intensité (400a, b), éventuellement dont le bruit a été filtré, un premier pic (402,a,b) et un second pic (462,a,b) présentent les amplitudes maximales les plus élevées par rapport à d'autres régions, dans lequel le premier pic (402,a,b) correspond au premier bord avant (202, a,b) et le second pic (462,a,b) correspond au second bord avant (262a, b,c).

5. Procédé selon l'une quelconque des revendications précédentes, dans lequel l'adhésivité est déterminée à partir du taux de gonflement et du taux de pénétration, dans lequel un échantillon de film présentant une adhésivité plus élevée présente un taux de gonflement et un taux de pénétration plus élevés par rapport à un échantillon de film présentant une adhésivité plus faible.

6. Procédé selon l'une quelconque des revendications précédentes, dans lequel la détermination à partir des paramètres d'expansion, de l'adhésivité de l'échantillon de film (108) comprend les étapes consistant à :
- recevoir comme entrée un modèle prédictif entraîné de l'ensemble des paramètres d'expansion,
- produire à partir du modèle prédictif entraîné l'adhésivité de l'échantillon de film (108),
dans lequel le modèle a été entraîné à l'aide d'un ensemble de données d'apprentissage comprenant le taux de gonflement, le taux de pénétration et l'adhésivité mesurée expérimentalement, et
- une entrée d'apprentissage dans le modèle prédictif est le taux de gonflement, le taux de pénétration, et une sortie d'apprentissage est l'adhésivité prédite,
- l'adhésivité prédite pendant l'apprentissage est comparée à l'adhésivité mesurée expérimentalement, et
- le modèle prédictif est ajusté pendant l'apprentissage de sorte que l'adhésivité prédite émise se rapproche de l'adhésivité mesurée expérimentalement de l'ensemble de données d'apprentissage.

7. Procédé selon l'une quelconque des revendications précédentes, dans lequel le taux de gonflement et le taux de pénétration sont comparés à des taux de gonflement connus et à des taux de pénétration connus d'échantillons présentant des adhésivités connues, déterminant ainsi l'adhésivité de l'échantillon de film (108).

8. Procédé selon l'une quelconque des revendications précédentes, dans lequel
- le taux de gonflement est représenté comme première fonction exponentielle ajustée à la position (*p1*) du premier bord avant (202, a, b, c) sur les différents points temporels,
- le taux de pénétration est représenté comme seconde fonction exponentielle ajustée à la position (*p2*) du second bord avant (262, a, b, c) sur les différents points temporels,
dans lequel la première fonction exponentielle et la seconde fonction exponentielle sont comparées à des première et seconde fonctions exponentielles connues d'échantillons présentant des adhésivités connues, déterminant ainsi l'adhésivité de l'échantillon de film (108).

9. Procédé selon l'une quelconque des revendications précédentes, dans lequel le film est un film d'adhérence humide contenant un ou plusieurs parmi l'acrylonitrile butadiène styrène, le polybutadiène, les polycarbonates, les polysulfones, le téréphtalate de polyéthylène, le poly(méthacrylate de méthyle), le polystyrène, le polychlorure de vinyle, le polyalcool de vinyle-eau, le polyalcool de vinyle, de préférence contenant du polyalcool de vinyle.

10. Procédé selon l'une quelconque des revendications précédentes, dans lequel le procédé est mis en œuvre par ordinateur, et dans lequel la mesure de l'ensemble de paramètres d'expansion comprend :
- la réception d'un ensemble de données d'image comprenant une pluralité d'images optiques (200, a, b, c) d'au moins une partie d'un bord latéral droit (102) de l'échantillon de film (108), chaque image optique (200, a, b, c) étant capturée à un moment différent pendant la mise en contact du bord latéral droit (102) de l'échantillon de film (108) avec un liquide aqueux (160),
- l'identification dans chaque image optique (200, a, b, c) ou une partie d'image (200', a, b, c) de celle-ci d'un premier bord avant (202, a, b, c) correspondant au bord latéral droit (102), et d'un second bord avant (262a, b, c) correspondant à un front liquide (162) du liquide aqueux (160) pénétrant dans l'échantillon de film (108) ; et
dans lequel le taux de gonflement est déterminé à partir d'un changement de position (*p1*) du premier bord avant (202, a, b, c) sur les différents points temporels, et le taux de pénétration est déterminé à partir d'un changement de position (*p2*) du second bord avant (262a, b, c) sur les différents points temporels,
- la détermination à partir des paramètres d'expansion, de l'adhésivité de l'échantillon de film (108).

11. Procédé selon l'une quelconque des revendications 1 à 9, dans lequel le procédé est mis en œuvre par ordinateur, et dans lequel la mesure de l'ensemble des paramètres d'expansion comprend :
- l'envoi, à un processeur distant, d'un ensemble de données d'images comprenant une pluralité d'images optiques (200, a, b, c) d'au moins une partie d'un bord latéral droit (102) de l'échantillon de film (108), chaque image optique (200, a, b, c) étant capturée à un point temporel différent pendant la mise en contact du bord latéral droit (102) de l'échantillon de film (108) avec un un liquide aqueux (160) ;
- la réception depuis le processeur distant, de l'adhésivité de l'échantillon de film, dans lequel l'adhésivité de l'échantillon de film a été déterminée par :
- l'identification dans chaque image optique (200, a, b, c) d'un premier bord avant (202, a, b, c) correspondant au bord latéral droit (102), et d'un second bord avant (262a, b, c) correspondant à un front liquide (162) du liquide aqueux (160) pénétrant dans l'échantillon de film (108) ; et
dans lequel le taux de gonflement est déterminé à partir d'un changement de position (*p1*) du premier bord avant (202, a, b, c) sur les différents points temporels, et le taux de pénétration est déterminé à partir d'un changement de position (*p2*) du second bord avant (262a, b, c) sur les différents points temporels,
- la détermination à partir des paramètres d'expansion, de l'adhésivité de l'échantillon de film (108).
